# EUROPEAN PATENT APPLICATION

(11) **EP 4 342 406 A2**
(43) Date of publication of application: **27.03.2024**
(21) Application number: 23198748.8
(22) Date of filing: 21.09.2023
(51) Int. Cl.: A61B 18/14, A61M 25/01

(54) **TWO-SEGMENT DEFLECTION CATHETER WITH SIDE EXIT GUIDEWIRE LUMEN**

(30) Priority: 22.09.2022 US 202263409050 P; 15.09.2023 US 202318468442
(71) Applicant: Biosense Webster (Israel) Ltd., Yokneam 2066717 (IL)
(72) Inventor: TOH, Eajer, Irvine, 92618 (US); LOVEJOY, Erica E., Irvine, 92618 (US); VAN, Jennifer, Irvine, 92618 (US)
(74) Representative: Carpmaels & Ransford LLP

(57) **Abstract**

An apparatus includes a body, a catheter, and a deflection assembly. The catheter includes a proximal segment defining a first longitudinal axis, a medial segment defining a second longitudinal axis, and a distal segment. The deflection assembly is configured to deflect the medial segment away from the first longitudinal axis and to deflect the distal segment away from the second longitudinal axis. The deflection assembly includes first and second input members associated with the body. The deflection assembly also includes a first translating assembly coupled to the medial segment. The first input member is configured to drive the first translating assembly to deflect the medial segment away from the first longitudinal axis. The deflection assembly further includes a second translating assembly coupled to the distal segment. The second input member is configured to drive the second translating assembly to deflect the distal segment away from the second longitudinal axis.

## Description

### CROSS-REFERENCE TO RELATED APPLICATION(S)

The present application claims priority to and the benefit of U.S. Provisional Patent Application No. 63/409,050, filed September 22, 2022, the entire content of which is incorporated herein by reference.

### FIELD OF INVENTION

This invention relates to catheters with deflectable shafts, in particular, catheters with deflectable shafts configured to receive other elongated devices, for example, a probe, catheter or guidewire to be passed therethrough.

### BACKGROUND

Cardiac arrhythmias, such as atrial fibrillation, occur when regions of cardiac tissue abnormally conduct electric signals. Procedures for treating arrhythmia include surgically disrupting the conducting pathway for such signals. By selectively ablating cardiac tissue by application of electrical energy (e.g., radiofrequency (AC type) or pulsed field (DC type) energy), it may be possible to cease or modify the propagation of unwanted electrical signals from one portion of the heart to another. The ablation process may provide a barrier to unwanted electrical pathways by creating electrically insulative lesions or scar tissue that effectively block communication of aberrant electrical signals across the tissue.

In some procedures, a catheter with one or more electrodes may be used to provide ablation within the cardiovascular system. The catheter may be inserted into a major vein or artery (e.g., the femoral artery) and then advanced to position the electrodes within the heart or in a cardiovascular structure adjacent to the heart (e.g., the pulmonary vein). The one or more electrodes may be placed in contact with cardiac tissue or other vascular tissue and then activated with electrical energy to thereby ablate the contacted tissue. In some cases, the electrodes may be bipolar. In some other cases, a monopolar electrode may be used in conjunction with a ground pad or other reference electrode that is in contact with the patient. Irrigation may be used to draw heat from ablating components of an ablation catheter; and to prevent the formation of blood clots near the ablation site.

Examples of ablation catheters are described in U.S. Pub. No. 2013/0030426, entitled "Integrated Ablation System using Catheter with Multiple Irrigation Lumens," published January 31, 2013, the disclosure of which is incorporated by reference herein, in its entirety; U.S. Pub. No. 2017/0312022, entitled "Irrigated Balloon Catheter with Flexible Circuit Electrode Assembly," published November 2, 2017, the disclosure of which is incorporated by reference herein, in its entirety; U.S. Pub. No. 2018/0071017, entitled "Ablation Catheter with a Flexible Printed Circuit Board," published March 15, 2018, the disclosure of which is incorporated by reference herein, in its entirety; U.S. Pub. No. 2018/0056038, entitled "Catheter with Bipole Electrode Spacer and Related Methods," published March 1, 2018, the disclosure of which is incorporated by reference herein, in its entirety; U.S. Pat. No. 10,130,422, entitled "Catheter with Soft Distal Tip for Mapping and Ablating Tubular Region," issued November 20, 2018, the disclosure of which is incorporated by reference herein, in its entirety; U.S. Pat. No. 8,956,353, entitled "Electrode Irrigation Using Micro-Jets," issued February 17, 2015, the disclosure of which is incorporated by reference herein, in its entirety; and U.S. Pat. No. 9,801,585, entitled "Electrocardiogram Noise Reduction," issued October 31, 2017, the disclosure of which is incorporated by reference herein, in its entirety.

Some catheter ablation procedures may be performed after using electrophysiology (EP) mapping to identify tissue regions that should be targeted for ablation. Such EP mapping may include the use of sensing electrodes on a catheter (e.g., the same catheter that is used to perform the ablation or a dedicated mapping catheter). Such sensing electrodes may monitor electrical signals emanating from conductive endocardial tissues to pinpoint the location of aberrant conductive tissue sites that are responsible for the arrhythmia. Examples of an EP mapping system are described in U.S. Pat. No. 5,738,096, entitled "Cardiac Electromechanics," issued April 14, 1998, the disclosure of which is incorporated by reference herein, in its entirety. Examples of EP mapping catheters are described in U.S. Pat. No. 9,907,480, entitled "Catheter Spine Assembly with Closely-Spaced Bipole Microelectrodes," issued March 6, 2018, the disclosure of which is incorporated by reference herein, in its entirety; U.S. Pat. No. 10,130,422, entitled "Catheter with Soft Distal Tip for Mapping and Ablating Tubular Region," issued November 20, 2018, the disclosure of which is incorporated by reference herein, in its entirety; and U.S. Pub. No. 2018/0056038, entitled "Catheter with Bipole Electrode Spacer and Related Methods," published March 1, 2018, the disclosure of which is incorporated by reference herein, in its entirety.

Some catheter procedures may be performed using an image guided surgery (IGS) system. The IGS system may enable the physician to visually track the location of the catheter within the patient, in relation to images of anatomical structures within the patient, in real time. Some systems may provide a combination of EP mapping and IGS functionalities, including the CARTO 3° system by Biosense Webster, Inc. of Irvine, California. Examples of catheters that are configured for use with an IGS system are disclosed in U.S. Pat. No. 9,480,416, entitled "Signal Transmission Using Catheter Braid Wires," issued November 1, 2016, the disclosure of which is incorporated by reference herein, in its entirety; and various other references that are cited herein.

While several catheter systems and methods have been made and used, it is believed that no one prior to the inventors has made or used the invention described, illustrated and claimed herein.

### SUMMARY OF THE DISCLOSURE

In some embodiments, a steerable and deflectable catheter of a catheter assembly may initially be inserted into a major vein or artery (e.g., the femoral vein) of the patient (PA) and then advanced distally along the vein or artery to position medial and distal segments of the catheter within target regions of a patient's heart with unique anatomical features. The medial segment may be deflected in a first direction that may also deflect the distal segment. The distal segment may be further advanced and deflected in a second direction. Deflection adjustments may be made to both the medial and distal segments to firmly secure catheter in place in the patient's heart, such as by frictional engagement with selected anatomical features in effectively providing a mechanical ground for the catheter relative to the heart.

After the catheter is suitably anchored, a guidewire may be advanced distally through a lumened shaft of the anchored catheter. The lumened shaft may be configured with a lateral opening through which a distal segment of the guidewire may be distally advanced to exit the shaft and reach additional target region of the heart with much more control than would otherwise occur in the absence of the anchored catheter.

In some embodiments, an apparatus includes a housing body and catheter shaft, the catheter shaft including a proximal segment defining a first longitudinal axis, a medial segment defining a second longitudinal axis, and a distal segment. Also included in the apparatus is a deflection assembly configured to deflect the medial segment away from the first longitudinal axis and to deflect the distal segment away from the second longitudinal axis. The deflection assembly includes a first input member, a second input member, a first translating assembly and a second translating assembly. The first translating assembly is coupled to the medial segment, the first input member being configured to drive the first translating assembly to deflect the medial segment away from the first longitudinal axis. The second translating assembly is coupled to the distal segment, the second input member being configured to drive the second translating assembly to deflect the distal segment away from the second longitudinal axis.

In some embodiments, the first and second translating assemblies includes first and second actuator cables, respectively.

In some embodiments, the first and second input members are configured to drive the first and second translating assemblies, respectively, independently of each other.

In some embodiments, the first and second input members include first and second lever arms, respectively, configured to rotate relative to the body about a drive axis.

In some embodiments, the drive axis is generally perpendicular with the longitudinal axis.

In some embodiments, the apparatus includes an end effector extending distally from the distal segment of the catheter.

In some embodiments, the end effector includes at least one ablative fluid port configured to deliver an ablative fluid to targeted tissue.

In some embodiments, the end effector includes a pair of occluding balloons configured to transition between respective non-expanded and expanded states, and the at least one ablative fluid port is longitudinally interposed between the pair of occluding balloons.

In some embodiments, the end effector includes at least one electrode.

In some embodiments, the at least one electrode is configured to emit electrical energy.

In some embodiments, the at least one electrode is configured to perform electrophysiology mapping.

In some embodiments, the end effector includes a position sensor.

In some embodiments, the catheter includes an inner lumen configured to slidably receive a guidewire, and the medial segment includes an opening configured to direct the guidewire out of the inner lumen.

In some embodiments, the opening is configured to direct the guidewire out of the inner lumen transversely to the second longitudinal axis.

In some embodiments, the opening is configured to direct the guidewire out of the inner lumen parallel to the second longitudinal axis.

In some embodiments, the opening is formed in a sidewall of the catheter shaft.

In some embodiments, the opening is formed in a sidewall of the medial segment of the shaft.

In some embodiments, the opening includes a distal slope surface configured in a sidewall of the catheter shaft.

In some embodiments, the catheter shaft includes a ramp disposed in the opening.

In some embodiments, the ramp extends at a nonperpendicular angle relative to the longitudinal axis of the medial segment.

In some embodiments, the ramp includes a Y configuration.

In some embodiments, the ramp includes first and second divergent prongs.

In some embodiments, the ramp includes first and second divergent prongs and the guidewire includes a ring stop configured to engage the first and second divergent prongs.

In some embodiments, the guidewire includes a predetermined pre-bent configuration when the guidewire is in a neutral form.

In some embodiments, the guidewire includes a shape memory construction.

In some embodiments, the guidewire includes a distal segment, a proximal segment and a transverse segment therebetween, the distal segment and the proximal segment being parallel but not coaxial when the guidewire is in neutral form.

In some embodiments, an apparatus includes a flexible catheter with a proximal segment defining a first longitudinal axis, a medial segment defining a second longitudinal axis, and a distal segment. The apparatus also includes an end effector and a deflection assembly. The end effector extends distally from the distal segment of the catheter and includes at least one ablative fluid port configured to deliver an ablative fluid to targeted tissue. The deflection assembly is configured to deflect the medial segment away from the first longitudinal axis, and to deflect the distal segment and the end effector away from the second longitudinal axis.

In some embodiments, the end effector includes a pair of occluding balloons configured to transition between respective non-expanded and expanded states, and the at least one ablative fluid port is longitudinally interposed between the pair of occluding balloons.

In some embodiments, an apparatus includes a flexible catheter, a guidewire and a deflection assembly. The flexible catheter includes a proximal segment, a medial segment, a distal segment and an inner lumen. The proximal segment defines a first longitudinal axis. The medial segment defines a second longitudinal axis, the medial segment including an opening. The inner lumen extends along the proximal and medial segments to the opening. The guidewire slidably disposed in the inner lumen, the opening configured to direct the guidewire out of the inner lumen. The deflection assembly is configured to deflect the medial segment away from the first longitudinal axis, and to deflect the distal segment away from the second longitudinal axis.

In some embodiments, the guidewire includes at least one electrode.

In some embodiments, the at least one electrode is configured to perform electrophysiology mapping.

### BRIEF DESCRIPTION OF THE DRAWINGS

The drawings and detailed description that follow are intended to be merely illustrative and are not intended to limit the scope of the invention as contemplated by the inventors.
FIG. 1 depicts a schematic view of a medical procedure in which a catheter of a catheter assembly is inserted in a patient;
FIG. 2 depicts a perspective view of the catheter assembly of FIG. 1, with additional components shown in schematic form;
FIG. 3 depicts a perspective view of a distal portion of the catheter of FIG. 1, with additional components shown in schematic form;
FIG. 4A depicts a top plan view of a handle and a deflection drive assembly of the catheter assembly of FIG. 1, where an elongated body of the deflection drive assembly is in a first rotational position relative to the handle;
FIG. 4B depicts a top plan view of the handle and the deflection drive assembly of FIG. 4A, where the elongated body of FIG. 4A is in a second rotational position relative to the handle;
FIG. 4C depicts a top plan view of the handle and the deflection drive assembly of FIG. 4A, where the elongated body of FIG. 4A is in a third rotational position relative to the handle;
FIG. 5A depicts a top plan view of the distal portion of the catheter of FIG. 1, with a portion of the outer sheath omitted to reveal internal components, where the distal portion of the catheter is in a non-deflected position associated with the first rotational position of the elongated body of FIG. 4A;
FIG. 5B depicts a top plan view of the distal portion of the catheter of FIG. 1, with a portion of the outer sheath omitted to reveal internal components, where the distal portion of the catheter is in a first deflected position associated with the second rotational position of the elongated body of FIG. 4B;
FIG. 5C depicts a top plan view of the distal portion of the catheter of FIG. 1, with a portion of the outer sheath omitted to reveal internal components, where the distal portion of the catheter is in a second deflected position associated with the third rotational position of the elongated body of FIG. 4C;
FIG. 6 depicts a perspective view of another example of a catheter assembly having a catheter for insertion into a patient, with additional components shown in schematic form;
FIG. 7A depicts a perspective view of a distal portion of the catheter of FIG. 6, showing occluding balloons of an end effector of the catheter assembly in non-expanded states, with additional components shown in schematic form;
FIG. 7B depicts a perspective view of the distal portion of the catheter of FIG. 6, showing the occluding balloons of the end effector of the catheter assembly in expanded states, with additional components shown in schematic form;
FIG. 8A depicts a perspective view of a medial portion of the catheter of FIG. 6, showing a guidewire of the catheter assembly in a retracted position relative to a lateral opening of the catheter;
FIG. 8B depicts a perspective view of the medial portion of the catheter of FIG. 6, showing the guidewire of the catheter assembly in an extended position relative to the lateral opening of the catheter;
FIG. 8C depicts a side elevation view of the medial portion of a catheter showing a guidewire of the catheter assembly in an extended position relative to the lateral opening of the catheter, according to an embodiment, wherein a third central longitudinal axis (L3) defined by a proximal segment of the guidewire is not parallel with the medial portion;
FIG. 8D depicts a side elevation view of a guidewire with a pre-bent and/or pre-curved configuration, according to an embodiment;
FIG. 8E depicts a top plan view of a lateral opening with a ramp, according to an embodiment;
FIG. 8F depicts a side elevation view of the medial portion of a catheter showing a guidewire of the catheter assembly in an extended position relative to the lateral opening of the catheter, according to an embodiment, wherein a third central longitudinal axis (L3) defined by a proximal segment of the guidewire is generally parallel with the medial portion;
FIG. 8G depicts a side elevation view of a guidewire with a pre-bent and/or precurved configuration, according to an embodiment;
FIG. 9 depicts a side elevation view of a distal portion of the guidewire of FIG. 8A, showing the guidewire in a bent and/or curved configuration;
FIG. 10 depicts a perspective view of a handle and a deflection drive assembly of the catheter assembly of FIG. 6;
FIG. 11A depicts a top plan view of the handle and the deflection drive assembly of FIG. 10, showing a pair of lever arms of the deflection drive assembly in unactuated rotational positions relative to the handle;
FIG. 11B depicts a top plan view of the handle and the deflection drive assembly of FIG. 10, showing one of the lever arms of the deflection drive assembly in the unactuated rotational position relative to the handle and the other of the lever arms of the deflection drive assembly in an actuated rotational position relative to the handle;
FIG. 11C depicts a top plan view of the handle and the deflection drive assembly of FIG. 10, showing both of the lever arms of the deflection drive assembly in actuated rotational positions relative to the handle;
FIG. 12A depicts a top plan view of the handle and the deflection drive assembly of FIG. 10, with a portion of the handle omitted to reveal internal components, showing both of the lever arms of the deflection drive assembly in the unactuated rotational positions relative to the handle;
FIG. 12B depicts a top plan view of the handle and the deflection drive assembly of FIG. 10, with a portion of the handle omitted to reveal internal components, showing one of the lever arms of the deflection drive assembly in the unactuated rotational position relative to the handle and the other of the lever arms of the deflection drive assembly in the actuated rotational position relative to the handle;
FIG. 12C depicts a top plan view of the handle and the deflection drive assembly of FIG. 10, with a portion of the handle omitted to reveal internal components, showing both of the lever arms of the deflection drive assembly in the actuated rotational positions relative to the handle;
FIG. 13A depicts a perspective view of the distal portion of the catheter of FIG. 6, showing medial and distal segments of the catheter in non-deflected positions associated with the unactuated rotational positions of the respective lever arms of FIG. 11A;
FIG. 13B depicts a perspective view of the distal portion of the catheter of FIG. 6, showing the medial segment of the catheter in a deflected position associated with the actuated rotational position of the respective lever arm of FIG. 11B;
FIG. 13C depicts a perspective view of the distal portion of the catheter of FIG. 6, showing the distal segment of the catheter in a deflected position associated with the actuated rotational position of the respective lever arm of FIG. 11C;
FIG. 13D depicts a perspective view of the distal portion of the catheter of FIG. 6, showing the medial and distal segments of the catheter in the respective deflected positions, and further showing the guidewire of FIG. 8A in the extended position relative to the lateral opening of the catheter;
FIG. 13E depicts a perspective view of the distal portion of the catheter of FIG. 6, showing the medial and distal segments of the catheter in the respective deflected positions and the guidewire of FIG. 8A in the extended position relative to the lateral opening of the catheter, and further showing the distal segment of the catheter in an out-of-plane deflected position;
FIG. 13F depicts an end cross-sectional view of a catheter shaft with a lumen and multiple actuating cables situated on selected diameters of the lumen;
FIG. 14 depicts a cross-sectional view of another example of a deflection drive assembly for use with the catheter assembly of FIG. 6;
FIG. 15A depicts a schematic view of the patient's heart, showing the medial and distal segments of the catheter of FIG. 6 positioned within the inferior vena cava;
FIG. 15B depicts a schematic view of the patient's heart, showing the distal segment of the catheter of FIG. 6 positioned within the coronary sinus;
FIG. 15C depicts a schematic view of the patient's heart, showing the distal segment of the catheter of FIG. 6 positioned within the oblique vein of the left atrium;
FIG. 15D depicts a schematic view of the patient's heart, showing the distal segment of the catheter of FIG. 6 frictionally engaged with the oblique vein of the left atrium and the medial segment of the catheter of FIG. 6 frictionally engaged with the coronary sinus to anchor the catheter relative to the heart, and further showing the guidewire of FIG. 8A in the extended position relative to the lateral opening of the catheter and positioned within the great cardiac vein;
FIG. 15E depicts a schematic view of the oblique vein of the left atrium, showing the occluding balloons of the end effector of the catheter assembly of FIG. 6 in the expanded states to sealingly engage the oblique vein of the left atrium for fluidly isolating a region of the oblique vein of the left atrium between the occluding balloons;
FIG. 15F depicts a schematic view of the oblique vein of the left atrium, showing the occluding balloons of the end effector of the catheter assembly of FIG. 6 in the expanded states to sealingly engage the oblique vein of the left atrium, and further showing delivery of ablative fluid to the fluidly isolated region of the oblique vein of the left atrium to ablate targeted tissue within the fluidly isolated region;
FIG. 15G depicts a schematic view of the oblique vein of the left atrium, showing the occluding balloons of the end effector of the catheter assembly of FIG. 6 in the expanded states to sealingly engage the oblique vein of the left atrium, and further showing evacuation of remaining ablative fluid from the fluidly isolated region of the oblique vein of the left atrium following ablation of the targeted tissue within the fluidly isolated region;
FIG. 15H depicts a schematic view of the oblique vein of the left atrium, showing the occluding balloons of the end effector of the catheter assembly of FIG. 6 in the non-expanded states to disengage the oblique vein of the left atrium; and
FIG. 16 depicts a schematic view of the patient's heart, showing the medial segment of the catheter of FIG. 6 positioned within the right atrium and the distal segment of the catheter of FIG. 6 positioned within the right ventricular outflow tract, and further showing the guidewire of FIG. 8A in the extended position relative to the lateral opening of the catheter and positioned within the right ventricle.

### DETAILED DESCRIPTION FOR MODES OF CARRYING OUT THE INVENTION

The following description of certain examples of the invention should not be used to limit the scope of the present invention but should be read with reference to the drawings. The drawings, which are not necessarily to scale, depict selected embodiments and are not intended to limit the scope of the invention. The detailed description illustrates by way of example, not by way of limitation, the principles of the invention. Other examples, features, aspects, embodiments, and advantages of the invention will become apparent to those skilled in the art from the following description, which is by way of illustration, one of the best modes contemplated for carrying out the invention. As will be realized, the invention is capable of other different or equivalent aspects, all without departing from the invention. Accordingly, the drawings and descriptions should be regarded as illustrative in nature and not restrictive.

Any one or more of the teachings, expressions, versions, examples, etc. described herein may be combined with any one or more of the other teachings, expressions, versions, examples, etc. that are described herein. The following-described teachings, expressions, versions, examples, etc. should therefore not be viewed in isolation relative to each other. Various suitable ways in which the teachings herein may be combined will be readily apparent to those skilled in the art in view of the teachings herein. Such modifications and variations are intended to be included within the scope of the claims.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 70% to 110%. In addition, as used herein, the terms "patient," "host," "user," and "subject" refer to any human or animal subject and are not intended to limit the systems or methods to human use, although use of the subject invention in a human patient represents a preferred embodiment.

### I. Overview of Example of a Catheter System

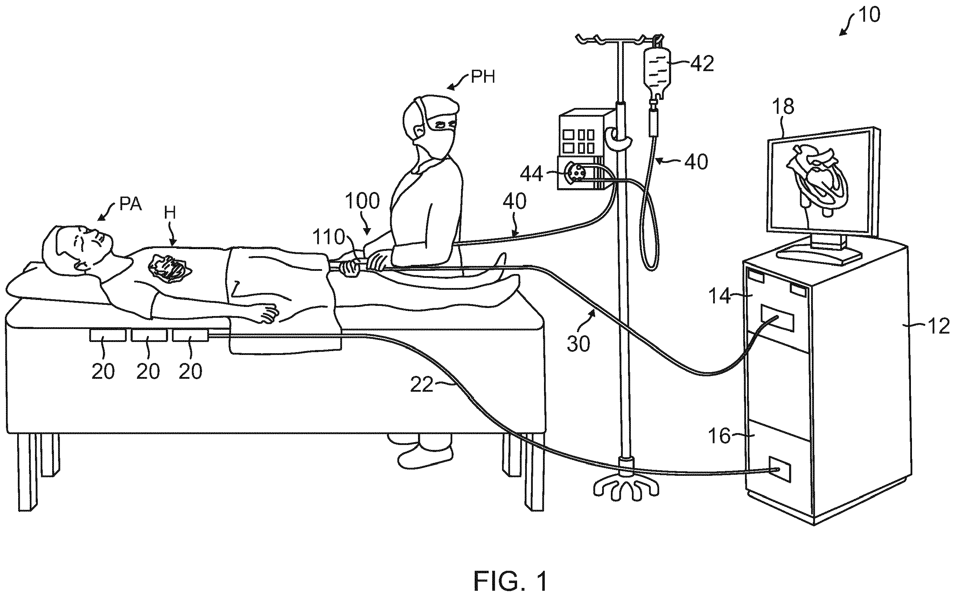

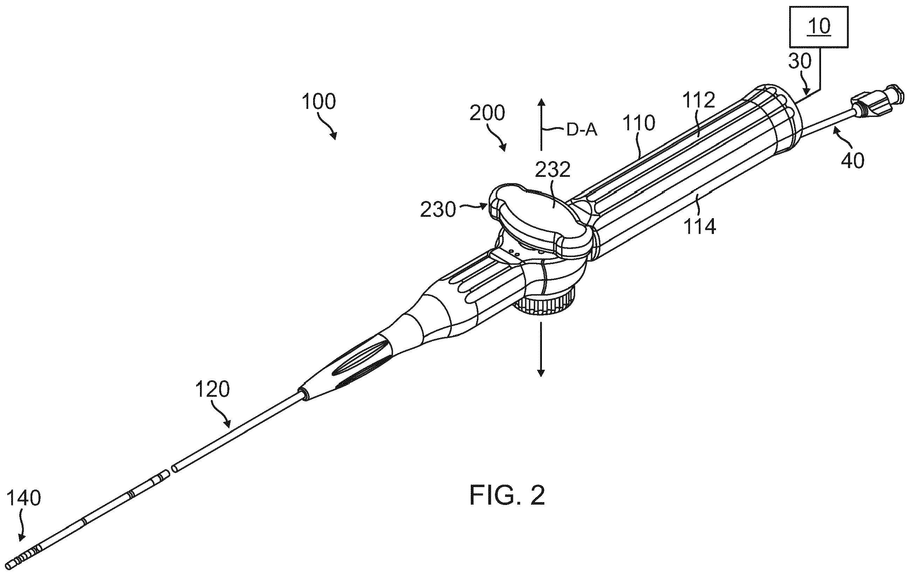

FIG. 1 shows an exemplary medical procedure and associated components of a cardiac catheter system that may be used to provide EP mapping or cardiac ablation as referred to above. In particular, FIG. 1 shows a physician (PH) grasping a handle (110) of a catheter assembly (100), with an end effector (140) of a catheter (120) (shown in FIGS. 2-3 but not shown in FIG. 1) of catheter assembly (100) disposed in a patient (PA) to map potentials in tissue and/or ablate tissue in or near the heart (H) of the patient (PA). As shown in FIG. 2, catheter assembly (100) includes handle (110), catheter (120) extending distally from handle (110), end effector (140) located at a distal end of catheter (120), and a deflection drive assembly (200) associated with handle (110).

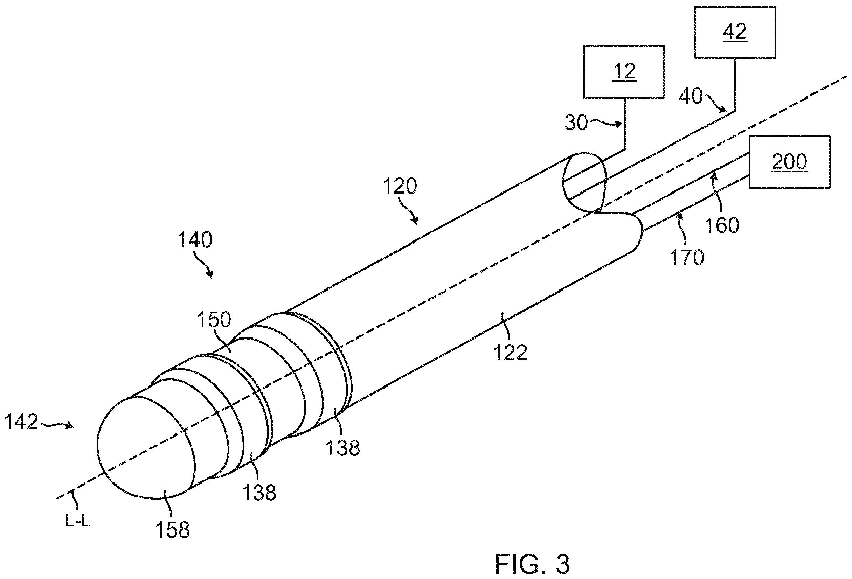

As will be described in greater detail below, end effector (140) may include various components configured to deliver electrical energy to targeted tissue sites, provide EP mapping functionality, track external forces imparted on end effector (140), track the location of end effector (140), and/or disperse fluid. As will also be described in greater detail below, deflection drive assembly (200) is configured to deflect end effector (140) and a distal portion of catheter (120) away from a central longitudinal axis (L-L) (FIGS. 3-5) defined by a proximal portion of catheter (120).

As shown in FIG. 3, catheter (120) includes an elongate flexible shaft (122), with end effector (140) being disposed at a distal end of shaft (122). End effector (140) and various components that are contained in shaft (122) will be described in greater detail below. Catheter assembly (100) is coupled with a guidance and drive system (10) via a cable (30). Catheter assembly (100) is also coupled with a fluid source (42) via a fluid conduit (40). A set of field generators (20) are positioned underneath the patient (PA) and are coupled with guidance and drive system (10) via another cable (22). Magnetic field generators (20) are merely optional.

As shown in FIG. 1, guidance and drive system (10) of the present example include a console (12) and a display (18). Console (12) includes a first driver module (14) and a second driver module (16). First driver module (14) is coupled with catheter assembly (100) via cable (30). First driver module (14) of the present example is operable to receive EP mapping signals obtained via ring-shaped electrodes (138) of end effector (140) as described in greater detail below. Console (12) includes a processor (not shown) that processes such EP mapping signals and thereby provides EP mapping as is known in the art.

In some variations, first driver module (14) may be further operable to provide electrical power to a distal tip member (142) of end effector (140) to thereby ablate tissue. Second driver module (16) is coupled with magnetic field generators (20) via cable (22). Second driver module (16) is operable to activate magnetic field generators (20) to generate an alternating magnetic field around the heart (H) of the patient (PA). For instance, field generators (20) may include coils that generate alternating magnetic fields in a predetermined working volume that contains the heart (H).

First driver module (14) may also be operable to receive position indicative signals from a navigation sensor assembly (not shown) in or near end effector (140). In such versions, the processor of console (12) is also operable to process the position indicative signals from the navigation sensor assembly to thereby determine the position of end effector (140) within the patient (PA). The navigation sensor assembly may include two or more coils on respective panels that are operable to generate signals that are indicative of the position and orientation of end effector (140) within the patient (PA). The coils are configured to generate electrical signals in response to the presence of an alternating electromagnetic field generated by magnetic field generators (20). Other components and techniques that may be used to generate real-time position data associated with end effector (140) may include wireless triangulation, acoustic tracking, optical tracking, inertial tracking, and the like. Alternatively, end effector (140) may lack a navigation sensor assembly.

Display (18) is coupled with the processor of console (12) and is operable to render images of patient anatomy. Such images may be based on a set of preoperatively or intraoperatively obtained images (e.g., a CT or MRI scan, 3-D map, etc.). The views of patient anatomy provided through display (18) may also change dynamically based on signals from the navigation sensor assembly of end effector (140). For instance, as end effector (140) of catheter (120) moves within the patient (PA), the corresponding position data from the navigation sensor assembly may cause the processor of console (12) to update the patient anatomy views in display (18) in real time to depict the regions of patient anatomy around end effector (140) as end effector (140) moves within the patient (PA). Moreover, the processor of console (12) may drive display (18) to show locations of aberrant conductive tissue sites, as detected via electrophysiological (EP) mapping with end effector (140) or as otherwise detected (e.g., using a dedicated EP mapping catheter, etc.). By way of example only, the processor of console (12) may drive display (18) to superimpose the locations of aberrant conductive tissue sites on the images of the patient's anatomy, such as by superimposing an illuminated dot, a crosshair, or some other form of visual indication of aberrant conductive tissue sites.

The processor of console (12) may also drive display (18) to superimpose the current location of end effector (140) on the images of the patient's anatomy, such as by superimposing an illuminated dot, a crosshair, a graphical representation of end effector (140), or some other form of visual indication. Such a superimposed visual indication may also move within the images of the patient anatomy on display (18) in real time as the physician moves end effector (140) within the patient (PA), thereby providing real-time visual feedback to the operator about the position of end effector (140) within the patient (PA) as end effector (140) moves within the patient (PA). The images provided through display (18) may thus effectively provide a video tracking the position of end effector (140) within a patient (PA), without necessarily having any optical instrumentation (i.e., cameras) viewing end effector (140). In the same view, display (18) may simultaneously visually indicate the locations of aberrant conductive tissue sites detected through EP mapping. The physician (PH) may thus view display (18) to observe the real time positioning of end effector (140) in relation to the mapped aberrant conductive tissue sites and in relation to images of the adjacent anatomical structures in the patient (PA).

Fluid source (42) of the present example includes a bag containing saline or some other suitable fluid. Conduit (40) includes a flexible tube that is further coupled with a pump (44), which is operable to selectively drive fluid from fluid source (42) to catheter assembly (100). In some variations, such fluid may be expelled through openings (not shown) of distal tip member (142) of end effector (140). Such fluid may be provided in any suitable fashion as will be apparent to those skilled in the art in view of the teachings herein.

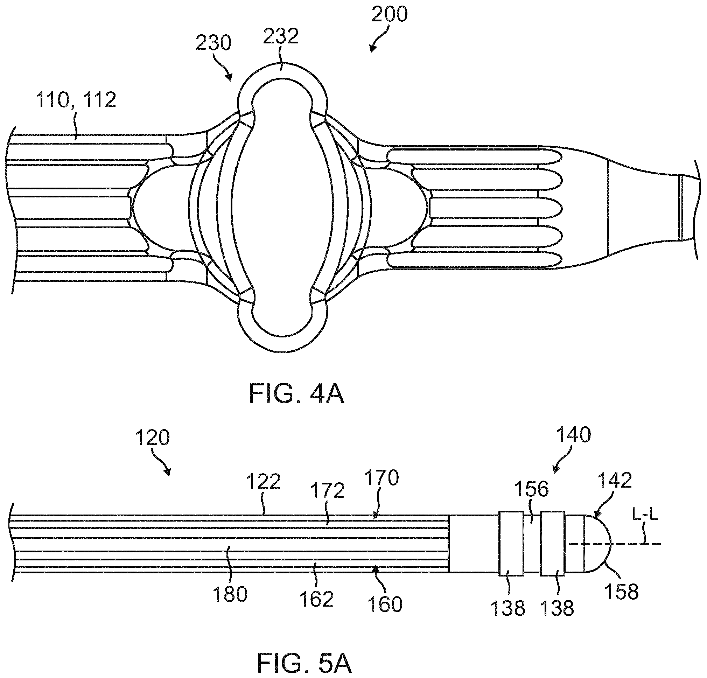

As mentioned above, end effector (140) may include various components configured to deliver electrical energy to targeted tissue sites, provide EP mapping functionality, track external forces imparted on end effector (140), track the location of end effector (140) within the patient (PA), and/or disperse fluid. FIG. 3 shows exemplary components of end effector (140), and other components of the distal portion of catheter (120), in greater detail. End effector (140) includes a distal tip member (142) secured to a distal end of shaft (122). A pair of actuator cables (160, 170) and a tube (180) (FIGS. 5A-5C) extend along the length of catheter (120) to reach end effector (140). Each of the foregoing components will be described in greater detail below. Flexible shaft (122) surrounds all of the foregoing components except for distal tip member (142).

As shown in FIGS. 3 and 5A-5C, distal tip member (142) of the present example includes a cylindraceous body (156) with a dome tip (158). Some versions of dome tip (158) may include an electrically conductive material, such as metal. In some such versions, dome tip (158) may also be operable to apply electrical energy to tissue to thereby ablate the tissue. Such electrical energy may be communicated from first driver module (14) to distal tip member (142) via cable (30). Distal tip member (142) may also include one or more thermocouples that are configured to provide temperature sensing capabilities. In addition, or alternatively, a plurality of openings (not shown) may be formed through cylindraceous body (156) and/or dome tip (158); and may be in communication with the hollow interior of distal tip member (142) to allow irrigation fluid to be communicated from the interior of distal tip member (142) out through cylindraceous body (156) and/or dome tip (158). In some variations, dome tip (158) does not deliver electrical energy and/or irrigation fluid.

As also shown in FIG. 3, distal tip member (142) of the present example also includes one or more EP mapping ring-shaped electrodes (138) mounted to cylindraceous body (156). In some other variations, cylindraceous body (156) is positioned distally in relation to electrodes (138). EP mapping electrodes (138) are configured to pick up electrical potentials from tissue that comes into contact with EP mapping electrodes (138). EP mapping electrodes (138) may thus be used to determine locations of aberrant electrical activity in tissue within a cardiovascular anatomical structure (e.g., pulmonary vein, etc.). Signals picked up by EP mapping electrodes (138) may be communicated through vias or other conductive structures, eventually reaching first driver module (14) of console (12) via cable (30). First driver module (14) may process the EP mapping signals and provide the physician (PH) with corresponding feedback indicating the locations of aberrant electrical activity in accordance with the teachings of various references cited herein.

In versions where cylindraceous body (156) is formed of an electrically conductive material to provide electrical energy for tissue ablation, an electrically insulating material may be interposed between cylindraceous body (156) and EP mapping electrodes (138) to thereby electrically isolate EP mapping electrodes (138) from cylindraceous body (156). EP mapping electrodes (138) may be constructed and operable in accordance with the teachings of various patent references cited herein. While two EP mapping electrodes (138) are shown, distal tip member (142) may include one or more than two EP mapping electrodes (138). Alternatively, distal tip member (142) may lack EP mapping electrodes (138) altogether.

As noted above, catheter assembly (100) includes a deflection drive assembly (200) configured to deflect end effector (140) away from the central longitudinal axis (L-L) defined by a proximal portion of catheter (120). Deflection drive assembly (200) of the present example incudes actuator cables (160, 170), a cable driver assembly (not shown), a rocker arm (230), and a load limiter assembly (not shown). As will be described in greater detail below, the physician (PA) may actuate rocker arm (230) relative to handle (110) such that the cable driver assembly actuates actuator cables (160, 170) in a simultaneous, longitudinally-opposing motion to selectively deflect end effector (140) laterally away from a longitudinal axis (L-L), thereby enabling the physician (PH) to actively steer end effector (140) within the patient (PA).

Selected portions of deflection drive assembly (200) are operatively coupled to handle (110). Handle (110) includes a first casing portion (112) and a second casing portion (114) together defining an internal cavity (not shown). Actuator cables (160, 170) include respective intermediary portions (162, 172), distal portions (not shown), and proximal end blocks (not shown). The proximal end blocks serve as a mechanical ground for actuator cables (160, 170). The distal end portions are coupled with end effector (140) to prevent actuator cables (160, 170) from being pulled proximally out of end effector (140). Suitable ways in which actuator cables (160, 170) may be coupled with (or anchored in or near) end effector (140) will be apparent to those skilled in the art in view of the teachings herein. Intermediary portions (162, 172) extend proximally from the distal portions, through elongate flexible shaft (122) of catheter (120) (as best shown in FIGS. 5A-5C), into the cable driver assembly, and terminate into proximal end blocks. Intermediary portions (162, 172) each wrap around a portion of the cable driver assembly such that movement of the cable driver assembly relative to handle (110) may actuate actuator cables (160, 170) simultaneously in opposite directions.

The cable driver assembly is rotationally coupled with handle (110). Specifically, cable driver (210) is configured to rotate about a drive axis such that suitable rotation of rocker arm (230) relative to handle (110) may drive rotation of cable driver (210) about a drive axis (D-A).

FIGS. 4A-5C show exemplary use of deflection drive assembly (200) to deflect end effector (140) and the distal portion of catheter (120) about central longitudinal axis (L-L). FIGS. 4A and 5A show various sections of catheter assembly (100) when end effector (140) is in a neutral, non-deflected position. FIG. 4A shows rocker arm (230) in a neutral rotational position relative to handle (110). When rocker arm (230) is in the first rotational position, the cable driver assembly is in a corresponding first rotation position such that actuator cables (160, 170) are in a first longitudinal position associated with end effector (140) being in the non-deflected position as shown in FIG. 5A.

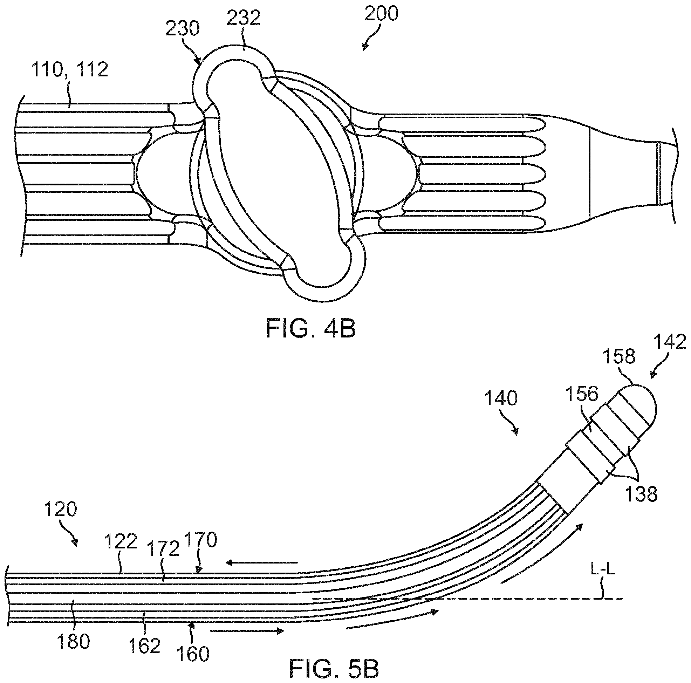

When the physician (PH) desires to deflect end effector (140) in a first direction relative to central longitudinal axis (L-L) to a first deflected position shown in FIG. 5B, the physician (PH) may rotate rocker arm (230) relative to handle (110) to the position shown in FIG. 4B. Rotation of rocker arm (230) to the rotational position shown in FIG. 4B drives the cable driver assembly into a corresponding rotational position such that actuator cable (170) is driven proximally and actuator cable (160) actuates distally, to thereby drive end effector (140) to bend to the position shown in FIG. 5B.

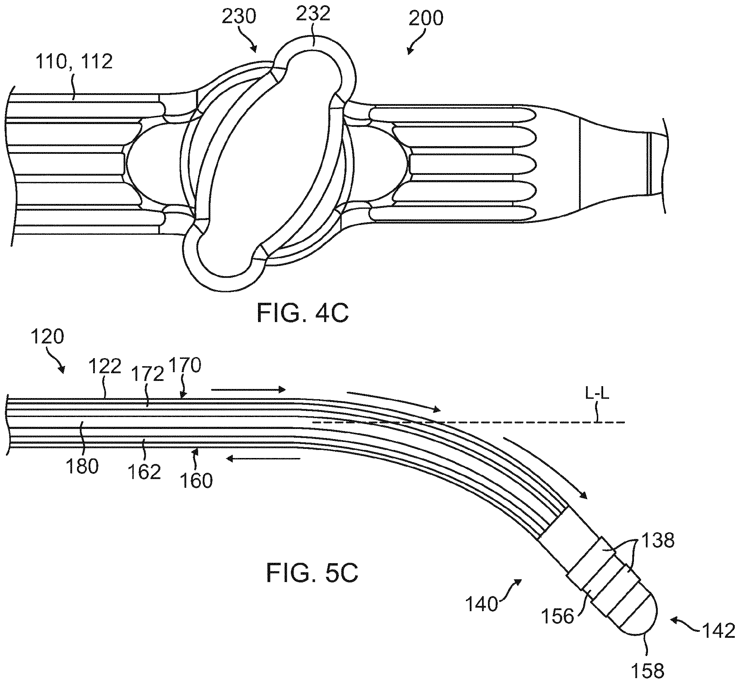

Similarly, when the physician (PH) desires to deflect end effector (140) in a second direction relative to central longitudinal axis (L-L) to a second deflected position shown in FIG. 5C, the physician (PH) may rotate rocker arm (230) relative to handle (110) to the position shown in FIG. 4C. Rotation of rocker arm (230) to the rotational position shown in FIG. 4C drives the cable driver assembly into a corresponding rotational position such that actuator cable (160) is driven proximally and actuator cable (170) actuates distally, to thereby drive end effector (140) to bend to the position shown in FIG. 5C.

Various other suitable mechanisms that may be used to drive actuator cables (160, 170) in a simultaneous, longitudinally-opposing fashion will be apparent to those skilled in the art in view of the teachings herein. In some versions, catheter assembly (100) may be configured and operable in accordance with one or more teachings of U.S. Pub. No. 2020/0405182, entitled "Catheter Deflection System with Deflection Load Limiter," published December 31, 2020, the disclosure of which is incorporated by reference herein.

### II. Example of a Catheter Assembly Having a Catheter with Two Deflectable Segments and a Lateral Opening

In some procedures, it may be desirable to securely stabilize a portion of catheter (120) in a substantially fixed position within the heart (H) to inhibit inadvertent movement (e.g., slipping) of catheter (120) that might otherwise result from patient cardioversion or inadvertent movement of handle (110) by the physician (PH), such as during EP mapping and/or tissue ablation. For example, it may be desirable to anchor a distal portion of catheter (120) within one or more accessory veins of the coronary sinus, such as the oblique vein of the left atrium (also referred to as the vein of Marshall). In some cases, it may be desirable to leverage the stabilization of the distal portion of catheter (120) within one region of the heart (H) to facilitate access by another device of one or more other regions of the heart (H) that may be difficult or impossible to access via catheter (120) itself (e.g., due to restrictions imposed by the outer diameter and/or bending radius of catheter (120)), such as to enable EP mapping of such other region(s). For example, it may be desirable to access the great cardiac vein (also referred to as the left coronary vein) with another catheter or a guidewire while a distal portion of catheter (120) remains anchored within the oblique vein of the left atrium. In addition, or alternatively, it may be desirable to ablate tissue within the region of the heart (H) in which the distal portion of catheter (120) is anchored via an ablative fluid such as ethanol (alcohol).

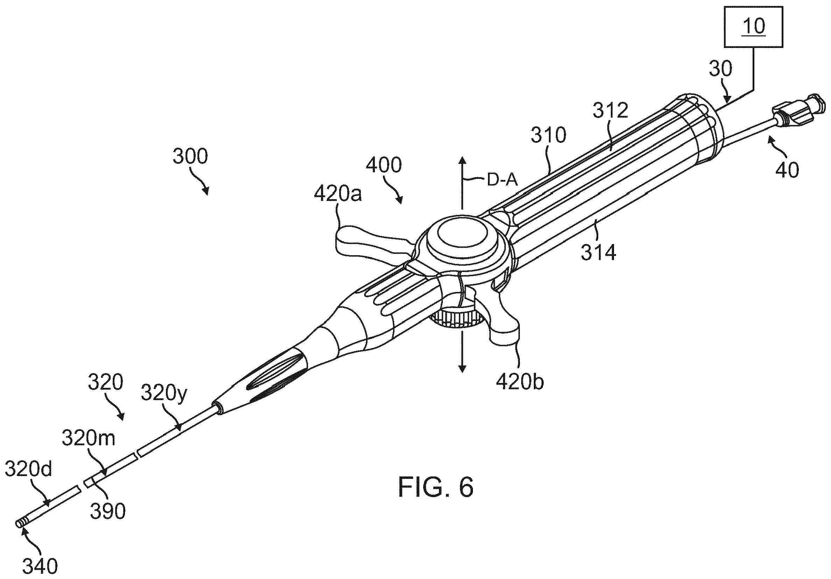

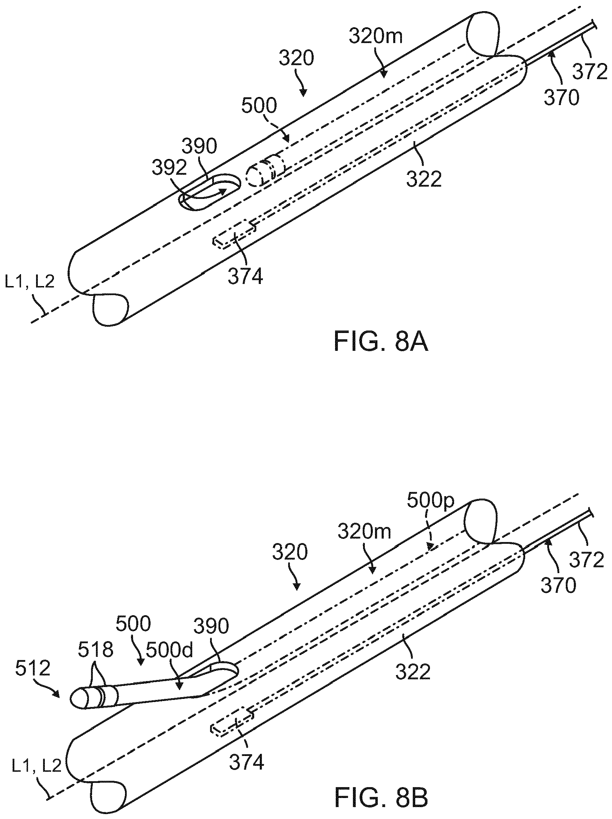

FIGS. 6-13E show an example of a catheter assembly (300) that may function in such a manner and that may be incorporated into the cardiac ablation catheter system of FIG. 1 in place of catheter assembly (100). Catheter assembly (300) may be configured and operable like catheter assembly (100) described above except as otherwise described below. In this regard, catheter assembly (300) includes a handle (310), a catheter (320) extending distally from handle (310), an end effector (340) located at a distal end of catheter (320), and a deflection drive assembly (400) associated with handle (310). In the example shown, catheter assembly (300) also includes a guidewire (500) (FIGS. 8A-9) slidably housed within catheter (320) such that guidewire (500) is extendable and retractable relative to catheter (320) between a retracted position (FIG. 8A) and an extended position (FIG. 8B). Handle (310) may include any suitable actuation features for driving translation of guidewire (500) relative to catheter (320), including but not limited to a slider, a pivoting rocker, a dial, etc.

As will be described in greater detail below, end effector (340) includes various components configured to provide EP mapping functionality and/or deliver ablative fluid to targeted tissue sites. In some versions, end effector (340) may additionally or alternatively include various components configured to deliver electrical energy to targeted tissue sites, track external forces imparted on end effector (340), track the location of end effector (340), and/or disperse irrigation fluid. For example, end effector (340) may be configured and operable in accordance with one or more teachings of U.S. Pub. No. 2020/0405182, entitled "Catheter Deflection System with Deflection Load Limiter," published December 31, 2020, the disclosure of which is incorporated by reference herein. In versions where end effector (340) includes one or more features that are operable to deliver electrical energy to tissue (e.g., to ablate the tissue), such electrical energy delivery features may be provided in addition to, or in lieu of, ablative fluid delivery features as described below. Moreover, some variations of end effector (340) may omit electrical energy delivery features and ablative fluid delivery features, such that neither type of feature is necessarily essential.

As will also be described in greater detail below, deflection drive assembly (400) is configured to deflect a medial segment (320m) of catheter (320) away from a first central longitudinal axis (L1) defined by a proximal segment (320p) of catheter (320); and to deflect end effector (340) and a distal segment (320d) of catheter (320) away from a second central longitudinal axis (L2) defined by medial segment (320m) of catheter (320). As will further be described in greater detail below, guidewire (500) is configured to provide EP mapping functionality in regions of the heart (H) that may not be readily accessible by end effector (140); and to assume a configuration in which a distal segment (500d) of guidewire (500) is deflected away from a third central longitudinal axis (L3) defined by a proximal segment (500p) of guidewire (500).

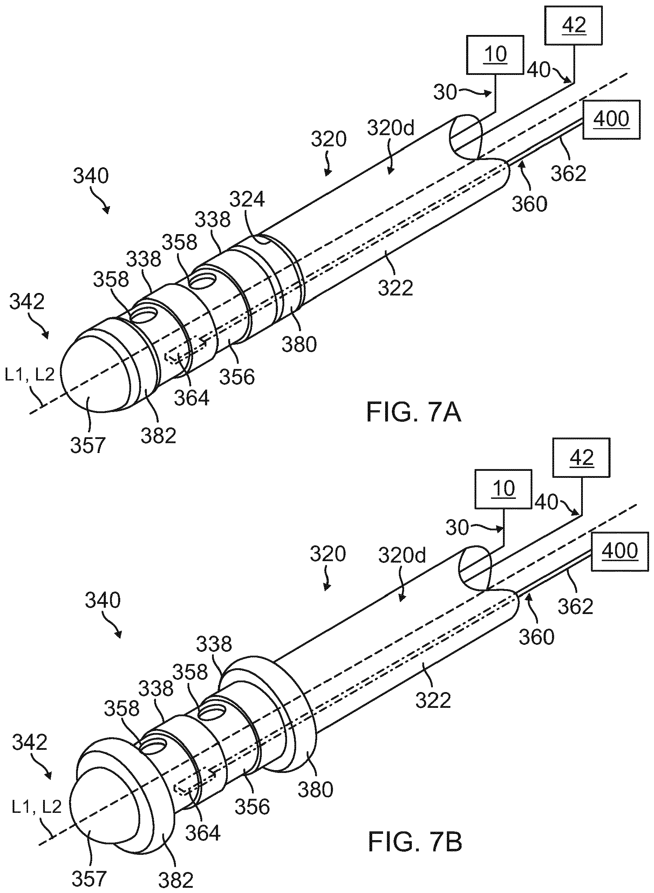

As shown in FIGS. 7A-7B, catheter (320) includes an elongate flexible shaft (322), with end effector (340) being disposed at a distal end (324) of shaft (322). End effector (340) and various components that are contained in/on shaft (322) will be described in greater detail below. Catheter assembly (300) is coupled with guidance and drive system (10) via cable (30). Catheter assembly (300) is also coupled with fluid source (42) via fluid conduit (40). In the present example, fluid source (42) includes a bag containing ethanol (alcohol) or some other suitable ablative fluid. Of course, fluid source (42) may instead take any other suitable form and contain any other suitable fluid.

As mentioned above, end effector (340) includes various components configured to provide EP mapping functionality and/or deliver ablative fluid to targeted tissue sites. With continuing reference to FIGS. 7A-7B, end effector (340) includes a distal tip member (342) secured to distal end (324) of shaft (322). A first actuator cable (360) and a tube (not shown) or other structure defining a lumen extend along the length of catheter (320) to reach end effector (340). Each of the foregoing components will be described in greater detail below. Flexible shaft (322) surrounds all of the foregoing components except for distal tip member (342).

As shown in FIGS. 7A-7B, distal tip member (342) of the present example includes a cylindraceous body (356) having a distal tip (357). Distal tip (357) is atraumatic and may have either a flat or dome shape, for example. In some other versions, distal tip (357) may have any other suitable atraumatic configuration. Distal tip member (342) is fixedly secured to shaft (322) at or near distal end (324) of shaft (322). For example, cylindraceous body (356) may include a proximal socket (not shown) that receives distal end (324) of shaft (322) and is fixedly secured to shaft (322). By way of example only, distal tip member (342) may be welded to shaft (322), soldered to shaft (322), adhered to shaft (322) using an adhesive or epoxy, press-fit onto shaft (322), and/or fixedly secured to shaft (322) in any other suitable fashion. In some versions, cylindraceous body (356) may be integrally formed together with a cylindrical sidewall of shaft (322) as a unitary (e.g., monolithic) piece.

In the example shown, a plurality of ablative fluid delivery ports (358) extend radially through cylindraceous body (356) and are in fluid communication with fluid conduit (40) via the tube extending along the length of catheter (320). Ports (358) thus allow ablative fluid to be communicated from fluid source (42) out through cylindraceous body (356). In addition, or alternatively, fluid delivery ports (358) may be in fluid communication with a source of suction (not shown). In some versions, some ports (358) may be in fluid communication with one of fluid conduit (40) or the source of suction, while other ports (358) may be in fluid communication with the other of fluid conduit (40) or the source of suction. While four ablative fluid delivery ports (358) are shown, distal tip member (342) may include any suitable number of fluid delivery ports (358). For example, a large quantity of minute fluid delivery ports (358) may be defined by one or more porous portions of cylindraceous body (356). Alternatively, distal tip member (142) may lack fluid delivery ports (358) altogether.

As shown in FIGS. 7A-7B, distal tip member (342) of the present example also includes proximal and distal occluding balloons (380, 382) mounted to cylindraceous body (356), such that each of the ablative fluid delivery ports (358) is longitudinally interposed between occluding balloons (380, 382). Occluding balloons (380, 382) are each in fluid communication with an inflation fluid source (not shown), which may include a bag containing saline or some other suitable inflation fluid, via one or more inflation fluid conduits (not shown). The inflation fluid may thus be communicated from the inflation fluid source to each occluding balloon (380, 382) to transition occluding balloons (380, 382) from respective non-expanded states (FIG. 7A) to respective expanded states (FIG. 7B); and back from each occluding balloon (380, 382) to the inflation fluid source to transition occluding balloons (380, 382) from the respective expanded states (FIG. 7B) to the respective non-expanded states (FIG. 7A). In some versions, occluding balloons (380, 382) may be in fluid communication with the inflation fluid source via a single shared inflation fluid conduit, such that occluding balloons (380, 382) may be configured to transition between the respective expanded and non-expanded states substantially simultaneously with each other. In some other versions, occluding balloons (380, 382) may be in fluid communication with the inflation fluid source via two separate inflation fluid conduits, such that occluding balloons (380, 382) may be configured to transition between the respective expanded and non-expanded states independently of each other. In still other versions, distal tip member (142) may lack occluding balloons (380, 382) altogether.

When in the respective expanded states, occluding balloons (380, 382) may be configured to sealingly engage corresponding portions of an inner surface of an anatomical structure, such as a lumen. For example, occluding balloons (380, 382) may be configured to sealingly engage corresponding portions of an inner surface of a blood vessel at or near the heart (H) (e.g., the oblique vein of the left atrium) when in the respective expanded states. In this manner, occluding balloons (380, 382) may be configured to cooperate with each other when in the respective expanded states to fluidly isolate the region of the blood vessel that extends longitudinally between occluding balloons (380, 382), in which ablative fluid delivery ports (358) are located, from other regions of the blood vessel, to thereby inhibit ablative fluid from inadvertently contacting non-targeted tissue outside of the fluidly isolated region of the blood vessel. Occluding balloons (380, 382) may thus promote controlled delivery of ablative fluid into the fluidly isolated region of the blood vessel.

As shown in FIGS. 7A-7B, distal tip member (342) of the present example also includes one or more EP mapping ring-shaped electrodes (338) mounted to cylindraceous body (356). EP mapping electrodes (338) are configured to pick up electrical potentials from tissue that comes into contact with EP mapping electrodes (338). EP mapping electrodes (338) may thus be used to determine locations of aberrant electrical activity in tissue within a cardiovascular anatomical structure (e.g., pulmonary vein, etc.). Signals picked up by EP mapping electrodes (338) may be communicated through vias or other conductive structures, eventually reaching first driver module (14) of console (12) via cable (30). First driver module (14) may process the EP mapping signals and provide the physician (PH) with corresponding feedback indicating the locations of aberrant electrical activity in accordance with the teachings of various references cited herein. EP mapping electrodes (338) may be constructed and operable in accordance with the teachings of various patent references cited herein. While two EP mapping electrodes (338) are shown, distal tip member (342) may include one or more than two EP mapping electrodes (338). Alternatively, distal tip member (342) may lack EP mapping electrodes (338) altogether.

In some variations, distal tip member (342) may be configured to deliver electrical energy to target tissue. Such electrical energy may include radiofrequency (AC type) electrical energy, pulsed field (DC type) electrical energy (e.g., irreversible electroporation, etc.), or some other form of electrical energy. In some such cases, cylindraceous body (356) may be formed of an electrically conductive material, such as metal. In some other variations in which distal tip member (342) is configured to deliver electrical energy to target tissue, distal tip member (342) may include one or more electrically conductive elements secured to cylindraceous body (356).

In some versions, catheter (320) includes at least one navigation sensor assembly (not shown), fixedly secured relative to shaft (322) and/or cylindraceous body (356), that is operable to generate signals (e.g., in response to the presence of an alternating electromagnetic field generated by field generators (20)) that are indicative of the position and orientation of shaft (322) and/or distal tip member (342) within the patient (PA), such as in a manner similar to that described above. Such a navigation sensor assembly may be configured as a single-axis sensor (SAS) (e.g., having a single electromagnetic coil wound about a single axis), as a dual-axis sensor (DAS) (e.g., having two electromagnetic coils wound about respective axes), or as a triple-axis sensor (TAS) (e.g., having three electromagnetic coils wound about respective axes). In addition, or alternatively, such a navigation sensor assembly may be configured as a flexible printed circuit board (PCB). By way of example only, such a navigation sensor assembly may be configured and operable in accordance with at least some of the teachings of U.S. Pub. No. 2022/0257093, entitled "Flexible Sensor Assembly for ENT Instrument," published August 18, 2022, the disclosure of which is incorporated by reference herein, in its entirety.

Referring now to FIGS. 8A-8B, catheter (320) includes a lateral opening (also referred to as a lateral aperture) (390) extending radially through the cylindrical sidewall of shaft (322) at or near a distal end of medial segment (320m) of catheter (320). Lateral opening (390) is thus proximal to end effector (340). Lateral opening (390) may define a distal end of an inner lumen (392) of shaft (322) in which guidewire (500) is slidably received, such that lateral opening (390) may be configured to direct guidewire (500) from inner lumen (392) of shaft (322) to an exterior of shaft (322). Thus, lateral opening (390) provides a pathway for guidewire (500) that is distally advanced through inner lumen (392) of shaft (322) to protrude laterally from the distal end of medial segment (320m) of catheter (320) or otherwise be exposed relative to the distal end of medial segment (320m). In other words, lateral opening (390) may facilitate side-exiting of guidewire (500) out of inner lumen (392) of catheter (320) in a direction transverse to the longitudinal axis (L2) defined by medial segment (320m), such that guidewire (500) may be extended and retracted relative to lateral opening (390) to transition guidewire (500) between a retracted position (FIG. 8A) and at least one extended position (FIG. 8B). As described in greater detail below, this may enable guidewire (500) to reach one or more regions of the heart (H) that may not be readily accessible by catheter (320), such as the great cardiac vein. A second actuator cable (370) extends along the length of proximal and medial segments (320p, 320m) of catheter (320) to reach the distal end of medial segment (320m) at or near lateral opening (390), as described in greater detail below.

In the example shown, medial and distal segments (320m, 320d) of catheter (320) have a same cross-dimension (e.g., diameter) as each other. Alternatively, distal segment (320d) may have a smaller cross-dimension than that of medial segment (320m). In some such cases, the cylindrical sidewall of shaft (322) may define a generally annular distally-facing surface at the distal end of medial segment (320m); and opening (390) may extend through the annular surface. In this manner, opening (390) may facilitate exiting of guidewire (500) out of inner lumen (392) of catheter (320) in a direction parallel to the longitudinal axis (L2) defined by medial segment (320m).

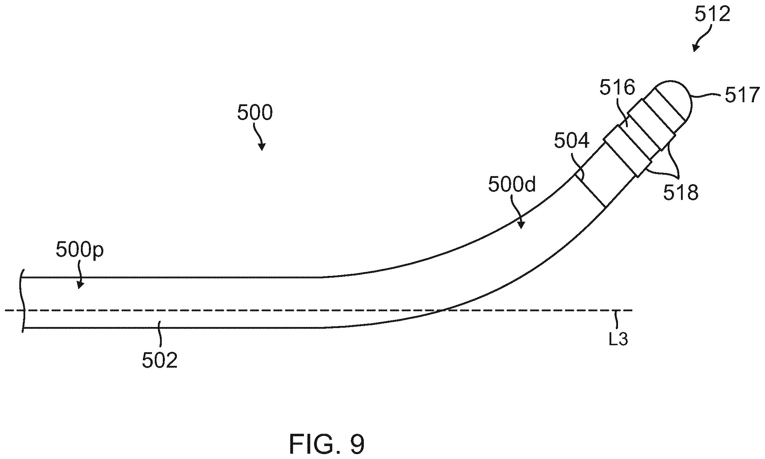

As best shown in FIG. 9, guidewire (500) of this example includes an elongate member (502) extending to a distal end (504). Elongate member (502) may include a shaft or a coil, for example. In cases where elongate member (502) includes a coil, the coil may be wrapped about a core wire (not shown), which may be secured relative to distal end (504) and prevent the coil from elongating when the coil is placed under tensile stress. Elongate member (502) is sized for insertion through inner lumen (392) of catheter (320). Catheter (320) may thus be used to assist in positioning a tip member (512) of guidewire (500) in relation to a target region of the heart (H). In this regard, guidewire (500) may include a locking member (not shown) configured to selectively affix guidewire (500) relative to catheter (320), such as to maintain a select portion of guidewire (500) extended relative to lateral opening (390) when guidewire (500) is in the at least one extended position (FIG. 8B).

In the example shown, distal segment (500d) of guidewire (500) is flexible, such that guidewire (500) may transition between a straight configuration (FIG. 8A) in which distal segment (500d) is coaxial with a proximal segment (500p) of guidewire (500), and a bent and/or curved configuration (FIGS. 8B and 9) in which distal segment (500d) and guidewire tip member (512) are deflected away from a third central longitudinal axis (L3) defined by proximal segment (500p). It will be appreciated that third longitudinal axis (L3) may be parallel to or colinear with second longitudinal axis (L2). In some versions, distal segment (500d) is pre-bent and/or pre-curved. In other words, distal segment (500d) may be pre-formed to define a bend and/or curve; or may otherwise be predisposed to define the bend and/or curve in the absence of external forces acting upon guidewire (500), such that guidewire (500) may be resiliently biased to assume the bent and/or curved configuration. As shown in FIGS. 8A and 8B, guidewire (500) may be configured to be restrained by inner lumen (392) of catheter (320) to assume the straight configuration while distal segment (500d) is slidably received within inner lumen (392) of catheter (320) (e.g., when guidewire (500) is in the retracted position), and to resiliently assume the bent and/or curved configuration in response to distal segment (500d) exiting inner lumen (392) of catheter (320) (e.g., when guidewire (500) is in the at least one extended position).

In addition to or in lieu of guidewire (500) having a resilient bias to urge distal segment (500d) to the bent and/or curved configuration when distal segment (500d) is exposed from catheter (320), inner lumen (392) may include a ramp and/or other structural feature that assists in urging distal segment (500d) laterally away from longitudinal axis (L2) as distal segment (500d) exits catheter (320) via lateral opening (390).

When guidewire (500) is in the bent and/or curved configuration, distal segment (500d) of guidewire (500) may be configured to promote advancement of guidewire (500) along a curved pathway defined by an anatomical structure, such as a lumen. For example, distal segment (500d) may be configured to promote advancement of guidewire (500) along a curved pathway defined by one or more blood vessels at or near the heart (H) (e.g., the coronary sinus and/or great cardiac vein) when in the bent and/or curved configuration. In addition, or alternatively, distal segment (500d) may be configured to reduce the risk of distal tip member (512) puncturing or otherwise imparting undesired trauma to such blood vessel(s) when in the bent and/or curved configuration.

In some other versions, distal segment (500d) may be predisposed to be straight relative to proximal segment (500p), such as in cases in which opening (390) is configured to facilitate exiting of guidewire (500) out of inner lumen (392) of catheter (320) in a direction parallel to the longitudinal axis (L2) defined by medial segment (320m). In other words, inner lumen (392) may include a ramp and/or other structural feature that assists in urging distal segment (500d) laterally away from longitudinal axis (L2) as distal segment (500d) exits catheter (320) via lateral opening (390), and distal segment (500d) may simply extend straight along that path as urged by the ramp and/or other structural feature of inner lumen.

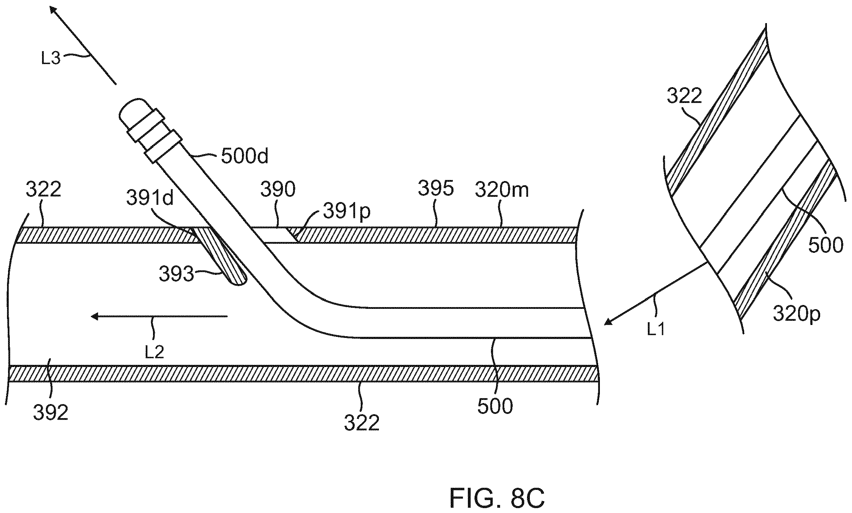

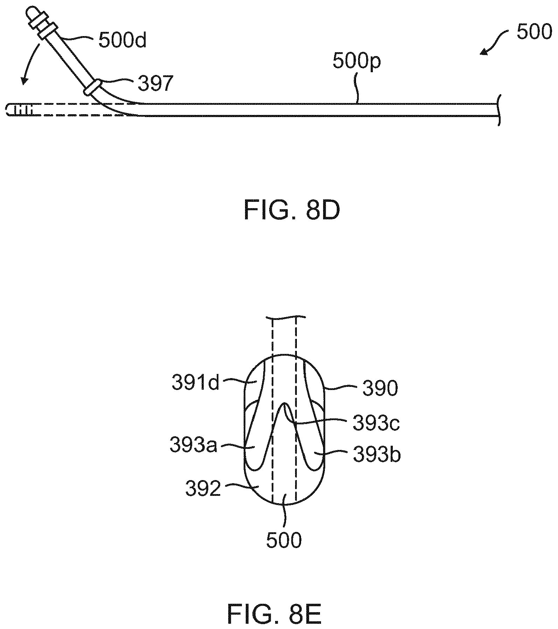

As shown in FIG. 8C, the lateral opening (390) configured in the sidewall of the catheter shaft (322) is defined by at least sloped portions of the surface that surrounds the lateral opening, including proximal slope surface (391d) and distal slope surface (391d) that form angles that are, respectively, lesser than and greater than 90 degrees with outer surface (395) of the shaft. The sloped surfaces, especially, the distal sloped surface (391d), facilitate the distal segment (500d) of the guidewire (500) to exit the lumen (392) of the shaft (322). In the embodiment depicted in FIG. 8C, a ramp (393) extends generally from the distal sloped surface (391d) into the lumen (392). The ramp (393) is sloped and assumes a similar angle as that of the distal sloped surface (391d) such that the guidewire (500), whether or not having a straight configuration or having a pre-bent and/or pre-curved configuration, can rest against and be guided by the ramp in exiting the lumen (392). The ramp may be shaped as a spade or a shovel. As shown in FIG. 8D, the guidewire may have shape-memory characteristics such that it has a pre-bent or pre-curved configuration wherein distal segment 500d extends at an angle relative to proximal segment 500p when the guidewire is in a neutral form (see solid lines) and can be resiliently straightened when exposed to external forces or constraints (see broken lines), for example, when passing through a lumen.

In other embodiments, as shown in FIG. 8E, the ramp (393) is "Y" shaped with two diverging prongs (393a, 393b) and a notch (393c) therebetween sized and shaped to support and can help stabilize the guidewire (500) in the lateral opening (390). In that regard, the guidewire (500) may include a ring stop 397 (see FIG. 8D) that is configured to abut the prongs (393a, 393b) of the ramp in generally affixing guidewire (500) relative to catheter (320), such as to maintain a portion of guidewire (500) extended relative to lateral opening (390).

The length of the ramp (393) or its prongs (393a, 393b) may depend on or be relative to the inner diameter of the lumen (392) such that the length may range between about 20% to about 60% of the inner diameter, so that the ramp is configured to better "catch" the distal end of the guidewire as it advances distally toward the lateral opening (390) and help direct and feed the distal segment (500d) toward the lateral opening (390).

As shown in FIG. 8C, the distal segment (500d) as it is further advanced distally and exits the lateral opening (390) defines a third central longitudinal axis (L3) that extends at an angle relative to the second central longitudinal axis (L2) defined by the medial segment (320m) of the shaft (322). Depending on the structure and configuration of the guidewire, including, for example, its flexibility or shape-memory, the distal segment (500d) and the third central longitudinal axis (L3) defined thereby may continue to extend along the angle relative to the second longitudinal axis (L2) or they extend parallel with the second longitudinal axis (L2).

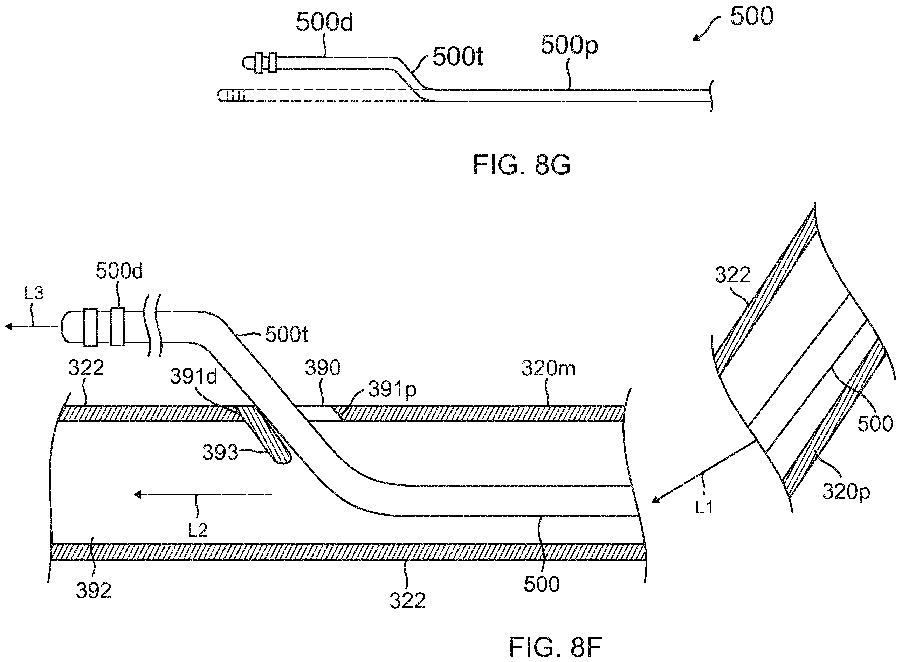

As shown in the embodiment of FIG. 8F and FIG. 8G, a guidewire may have shape-memory characteristics and be configured with a pre-bent or pre-curved configuration, such as "kink," where the distal segment 500d and the proximal segment 500p are connected by an off-angle transverse segment 500t. The segments 500d and 500p are parallel but not coaxial when the guidewire is in its neutral form (see solid lines) and can be resiliently straightened when subjected to external forces or constraints (see broken lines), for example, when passing through a lumen. The guidewire can therefore resiliently assume a straight configuration when being advanced distally through the lumen (392) and bend and flex as needed when the distal segment (500d) encounters the ramp (393). As the guidewire continues to be advanced distally, the ramp guides the distal segment (500d) through the lateral opening (390). After the transverse segment 500t enters the lateral opening (390), the distal segment (500d) and the third central longitudinal axis (L3) extend generally parallel with the second central longitudinal axis (L2). The location of the transverse segment 500t may be at any suitable location along the length of the guidewire. In instances where the transverse segment 500t is to remain in the lateral opening (390), the distal segment (500d) is of a sufficient length such that the distal end of the guidewire can reach the target tissue site.

As shown in FIG. 9, guidewire tip member (512) of the present example includes a cylindraceous body (516) having a distal tip (517). Distal tip (517) of the present example has a dome shape, such that distal tip (517) is atraumatic. In some other versions, distal tip (517) may have any other suitable atraumatic configuration. Guidewire tip member (512) is fixedly secured to elongate member (502) at or near distal end (504) of elongate member (502). For example, cylindraceous body (516) may include a proximal socket (not shown) that receives distal end (504) of elongate member (502) and is fixedly secured to elongate member (502). By way of example only, guidewire tip member (512) may be welded to elongate member (502), soldered to elongate member (502), adhered to elongate member (502) using an adhesive or epoxy, press-fit onto elongate member (502), and/or fixedly secured to elongate member (502) in any other suitable fashion.

As best shown in FIG. 9, guidewire tip member (512) of the present example also includes one or more EP mapping ring-shaped electrodes (518) mounted to body (516). EP mapping electrodes (518) are configured to pick up electrical potentials from tissue that comes into contact with EP mapping electrodes (518). EP mapping electrodes (518) may thus be used to determine locations of aberrant electrical activity in tissue within a cardiovascular anatomical structure (e.g., pulmonary vein, etc.). Signals picked up by EP mapping electrodes (518) may be communicated through vias or other conductive structures, eventually reaching first driver module (14) of console (12) via cable (30). First driver module (14) may process the EP mapping signals and provide the physician (PH) with corresponding feedback indicating the locations of aberrant electrical activity in accordance with the teachings of various references cited herein. EP mapping electrodes (518) may be constructed and operable in accordance with the teachings of various patent references cited herein. While two EP mapping electrodes (518) are shown, guidewire tip member (512) may include one or more than two EP mapping electrodes (518). Alternatively, guidewire tip member (512) may lack EP mapping electrodes (518) altogether.

In some variations, guidewire tip member (512) may be configured to deliver electrical energy to target tissue. Such electrical energy may include radiofrequency (AC type) electrical energy, pulsed field (DC type) electrical energy (e.g., irreversible electroporation, etc.), or some other form of electrical energy. In some such cases, cylindraceous body (516) may be formed of an electrically conductive material, such as metal. In some other variations in which guidewire tip member (512) is configured to deliver electrical energy to target tissue, guidewire tip member (512) may include one or more electrically conductive elements secured to cylindraceous body (516).

By way of example only, guidewire (500) may be configured and operable in accordance with at least some of the teachings of U.S. Pat. No. 11,213,344, entitled "Guidewire with Ablation and Coagulation Functionality," issued January 4, 2022, the disclosure of which is incorporated by reference herein, in its entirety; U.S. Pat. No. 10,603,472, entitled "Guidewires Having Improved Mechanical Strength and Electromagnetic Shielding," issued March 31, 2020, the disclosure of which is incorporated by reference herein, in its entirety; and/or U.S. Pub. No. 2021/0196370, entitled "Neurosurgery Guidewire with Integral Connector for Sensing and Applying Therapeutic Electrical Energy," published July 1, 2021, the disclosure of which is incorporated by reference herein, in its entirety.

In some versions, guidewire (500) includes at least one navigation sensor assembly (not shown) fixedly secured to elongate member (502) that is operable to generate signals (e.g., in response to the presence of an alternating electromagnetic field generated by field generators (20)) that are indicative of the position and orientation of elongate member (502) and/or tip member (512) within the patient (PA), such as in a manner similar to that described above. Such a navigation sensor assembly may be configured as a single-axis sensor (SAS) (e.g., having a single electromagnetic coil wound about a single axis), as a dual-axis sensor (DAS) (e.g., having two electromagnetic coils wound about respective axes), or as a triple-axis sensor (TAS) (e.g., having three electromagnetic coils wound about respective axes). In addition, or alternatively, such a navigation sensor assembly may be configured as a flexible printed circuit board (PCB). By way of example only, such a navigation sensor assembly may be configured and operable in accordance with at least some of the teachings of U.S. Pub. No. 2022/0257093, entitled "Flexible Sensor Assembly for ENT Instrument," published August 18, 2022, the disclosure of which is incorporated by reference herein, in its entirety.

While guidewire (500) has been described as being slidably received within inner lumen (392) of catheter (320) for accessing and providing EP mapping functionality to regions of the heart (H) that may not be readily accessed by catheter (320) itself, it will be appreciated that any other suitable type of device may be slidably received within inner lumen (392) of catheter (320) for any other suitable purposes. For example, a second catheter (not shown) may be incorporated into catheter assembly (300) in place of guidewire (500). Such a second catheter may include any one or more of the features described above with respect to guidewire (500).

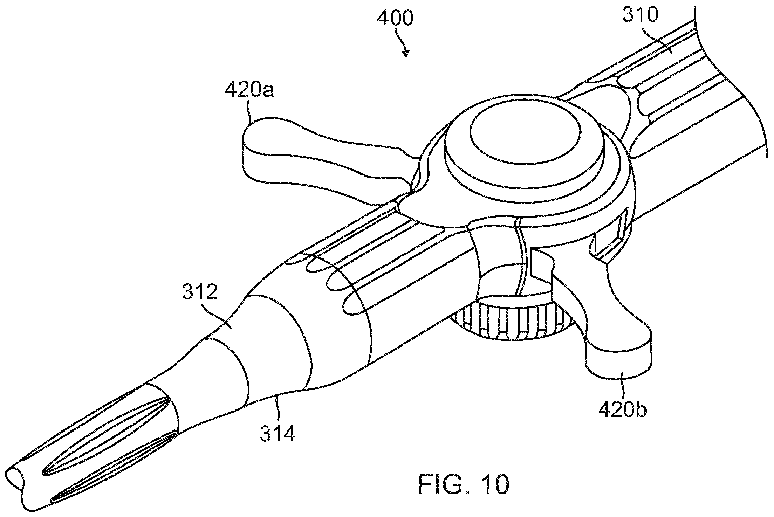

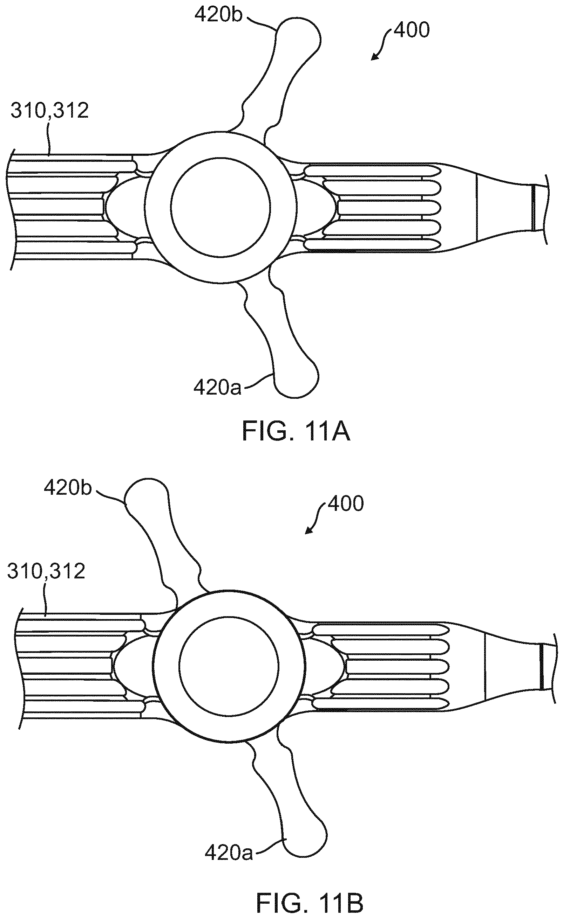

Referring now to FIG. 10, as noted above, catheter assembly (300) includes a deflection drive assembly (400) configured to deflect medial segment (320m) of catheter (320) away from first central longitudinal axis (L1) defined by proximal segment (320p) of catheter (320); and to deflect end effector (340) and distal segment (320d) of catheter (320) away from second central longitudinal axis (L2) defined by medial segment (320m) of catheter (320). It will be appreciated that first and second longitudinal axes (L1, L2) may be colinear with each other when medial segment (320m) is not deflected away from first longitudinal axis (L1). Deflection drive assembly (400) of the present example includes actuator cables (360, 370) and a cable driver assembly (410) including a pair of lever arms (420a, 420b). As will be described in greater detail below in connection with FIGS. 11A-13E, the physician (PA) may actuate lever arms (420a, 420b) relative to handle (310) independently of each other such that cable driver assembly (410) actuates the corresponding actuator cables (360, 370) to selectively deflect medial segment (320m) away from first central longitudinal axis (L1), and/or to selectively deflect end effector (340) and distal segment (320d) away from second central longitudinal axis (L2), thereby enabling the physician (PH) to actively steer end effector (340) within the patient (PA) and/or to securely anchor a distal portion of catheter (320) relative to one or more anatomical structures of the patient (PA).

Selected portions of deflection drive assembly (400) are operatively coupled to handle (310). Handle (310) includes a housing body with a first casing portion (312) and a second casing portion (314) together defining an internal cavity (302). First casing portion (312) defines a through hole (not shown) dimensioned to rotatably house a central body (412) of cable driver assembly (410). Lever arms (420a, 420b) may each suitably couple with central body (412) in accordance with description herein. Additionally, first casing portion (312) may include a pair of stops (not shown) located on opposite sides of the through hole and configured to limit the rotation of lever arms (420a, 420b) relative to handle (310).

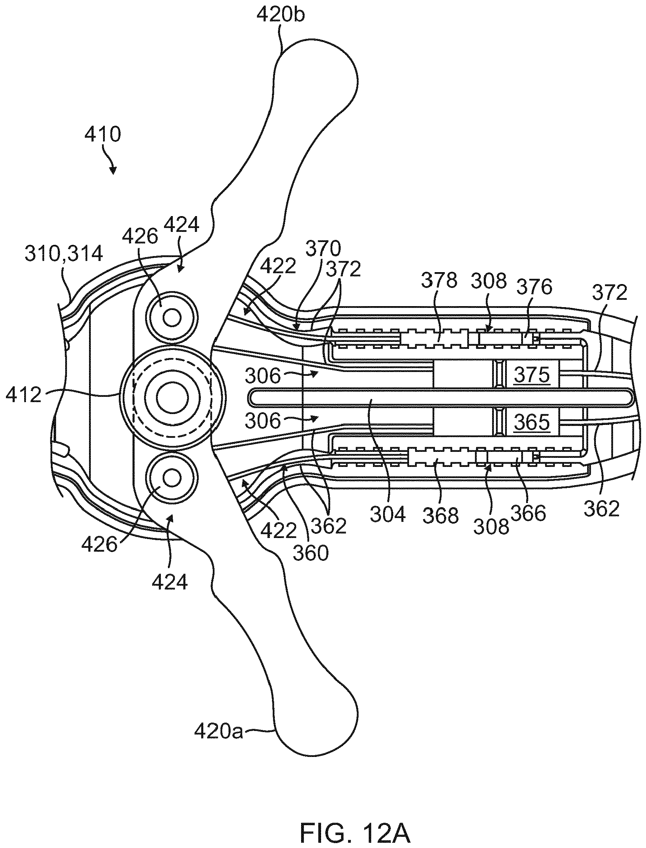

As best seen in FIGS. 12A-12B, an interior of second casing portion (314) includes a partition wall (304) and a pair of tension adjustment channels (308) located on opposite lateral sides of partition wall (304). Partition wall (304) and respective tension adjustment channels (308) together define a sliding channel (306). Each sliding channel (306) slidably houses a respective sliding body (365, 375). Sliding bodies (365, 375) are attached to respective actuator cables (360, 370). Sliding bodies (365, 375) and sliding channels (306) may together assist in guiding the translation of portions of actuator cables (360, 370) extending distally from sliding bodies (365, 375) in accordance with the description herein.

Tension adjustment channels (308) include a linear array of laterally extending, rectangular projections. Tension adjustment channels (308) are configured to receive respective tension blocks (368, 378), which also each have a complementary linear array of laterally extending rectangular projections. The complementary rectangular projections of tensions blocks (368, 378) and tension adjustment channels (308) are configured to longitudinally fix tension blocks (368, 378) relative to second casing portion (314). In other words, tension adjustment channels (308) are configured to receive tension blocks (368, 378) in a tongue-and-groove fashion to fix tension blocks (368, 378) relative to handle (310). Tension blocks (368, 378) may be selectively inserted along various suitable locations within adjustment channels (308) in order to serve as a mechanical ground for actuator cables (360, 370). Tension blocks (368, 378) may be inserted along various locations within adjustment channels (308) in order to adjust the tension within actuator cables (360, 370) to thereby accommodate for length variations of actuator cables (360, 370) due to various factors, such as manufacturing tolerance variations, deformation of actuator cables (360, 370), etc.

Actuator cables (360, 370) include respective intermediary portions (362, 372), distal portions (364, 374) (as best seen in FIGS. 7A-8B), and proximal end blocks (366, 376) (as best seen in FIGS. 12A-12C. As best seen in FIGS. 12A-12C, proximal end blocks (366, 376) are housed within tension adjustment channels (308) just distal to tension blocks (368, 378). Tension blocks (368, 378) therefore prevent proximal end blocks (366, 376) from actuating proximally within adjustment channels (308), thereby serving as a mechanical ground for actuator cables (360, 370). Proximal end blocks (366, 376) are fixed to respective intermediary portions (362, 372). Tension blocks (368, 378) define a through hole that intermediary portions (362, 372) extend through such that intermediary portions (362, 372) may extend from proximal end blocks (366, 376) through adjustment channels (308) in order to suitably couple with cable driver assembly (410). Alternatively, tension blocks (368, 378) and respective proximal end blocks (366, 376) may be formed of a single piece.

As best shown in FIGS. 7A-8B, respective distal portions (364, 374) have a larger outer cross dimension (e.g., width, thickness, diameter, etc.) than the outer diameter of respective intermediary portions (362, 372). First distal end portion (364) is coupled with end effector (340) to prevent actuator cable (360) from being pulled proximally out of end effector (340), while second distal end portion (374) is coupled with the distal end of medial segment (320m) of catheter (320) to prevent actuator cable (370) from being pulled proximally out of medial segment (320m). Suitable ways in which actuator cables (360, 370) may be coupled with (or anchored in or near) end effector (340) and medial segment (320m), respectively, will be apparent to those skilled in the art in view of the teachings herein.

With the distal end portion (364, 374) of each actuator cable (360, 370) anchored in the catheter (320) at a different location along the length of the catheter (320), the catheter advantageously exhibits a different respective deflection curve along the side of the catheter along which the respective actuator cable extends. As the respective deflection curve ends at the location of the respective anchor location, different respective anchor locations on different/opposing sides of the catheter provides asymmetric deflections, as needed or desired.

Intermediary portions (362, 372) extend proximally from distal portions (364, 374), through elongate flexible shaft (322) of catheter (320) (as best shown in FIGS. 7A-8B), into cable driver assembly (410), and terminate into proximal end blocks (366, 376) (as best shown in FIGS. 12A-12C). Intermediary portions (362, 372) may include various segments coupled to each other in order to extend between distal portions (364, 374) and proximal end blocks (366, 376). Various segments of intermediary portions (362, 372) may be coupled through any suitable means as would be apparent to one skilled in the art in view of the teachings herein. Intermediary portions (362, 372) each wrap around a corresponding portion of cable driver assembly (410) such that movement of the corresponding lever arm (420a, 420b) relative to handle (310) may actuate the respective actuator cable (360, 370).

As best seen in FIGS. 12A-12B, cable driver assembly (410) includes central body (412), with lever arms (420a, 420b) extending laterally from central body (412). Central body (412) is fixedly coupled with handle (310) while lever arms (420a, 420b) of cable driver assembly (410) are each rotationally coupled with handle (110) via central body (412). Specifically, lever arms (420a, 420b) are each configured to rotate about a drive axis (D-A) independently of each other. In some versions, central body (412) may define a pair of lateral slots (not shown) configured to receive respective portions of lever arms (420a, 420b).

Lever arms (420a, 420b) of cable driver assembly (410) are configured to couple with a respective actuator cable (360, 370) such that rotation of each lever arm (420a, 420b) about drive axis (D-A) actuates the corresponding actuator cable (360, 370) in accordance with the description herein. Each lever arm (420a, 420b) defines a cable recess (422) and a plug opening (424) extending into cable recess (422). Each cable recess (422) is dimensioned to receive the intermediary portions (362, 372) of the corresponding actuator cable (360, 370), while each plug opening (424) is dimensioned to receive a corresponding cable plug (426) such that each cable plug (426) actuates with the respective lever arm (420a, 420b). Each cable recess (422) is dimensioned to accommodate the corresponding cable plug (426) such that intermediary portions (362, 372) may each wrap around the corresponding cable plug (426), thereby suitably coupling intermediary portion (362, 372) of actuator cables (360, 370) with cable driver assembly (410). Cable plugs (426) each interact with the respective intermediary portion (362, 372) such that proximal movement of a cable plug (426) pulls the respective intermediary portion (362, 372) proximally.

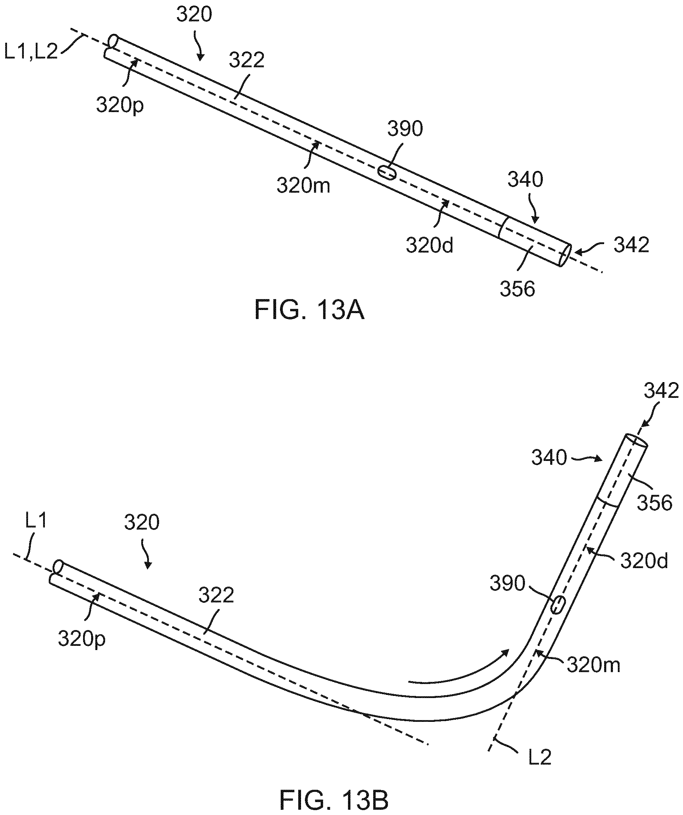

FIGS. 11A-13C show exemplary use of deflection drive assembly (400) to deflect medial segment (320m) of catheter (320) away from first central longitudinal axis (L1) defined by proximal segment (320p) of catheter (320), and to deflect end effector (340) and distal segment (320d) of catheter (320) away from second central longitudinal axis (L2) defined by medial segment (320m) of catheter (320). FIGS. 11A, 12A, and 13A show various sections of catheter assembly (300) when end effector (340) is in a neutral, non-deflected position. FIG. 11A shows both lever arms (420a, 420b) in a neutral rotational position relative to handle (110). As best shown in FIG. 12A, when both lever arms (420a, 420b) are in the neutral rotational position, sliding bodies (365, 375), and therefore actuator cables (360, 370), are each in a first longitudinal position associated with end effector (340) being in the non-deflected position as shown in FIG. 13A.

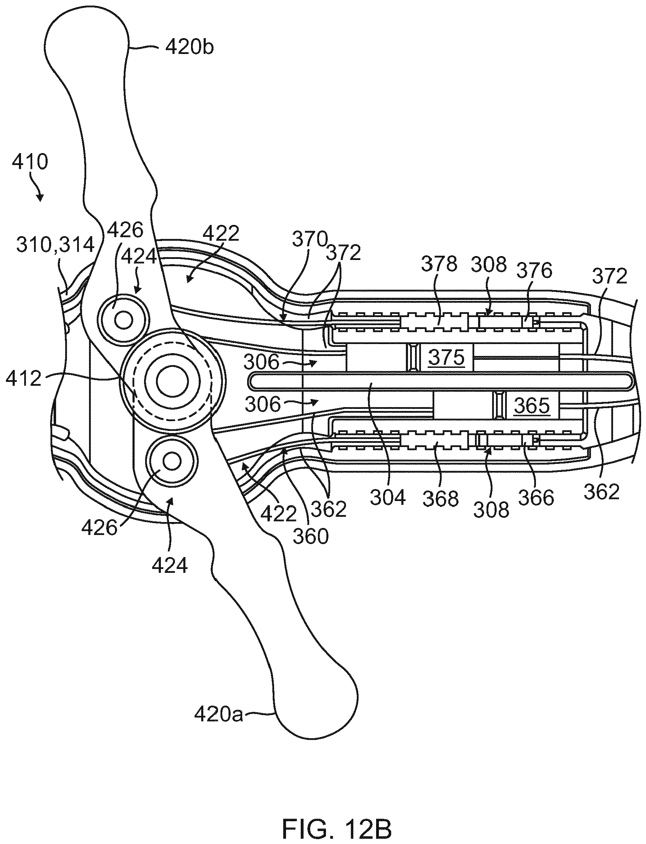

When the physician (PH) desires to deflect medial segment (320m) of catheter (320) in a first direction relative to first central longitudinal axis (L1) to a deflected state shown in FIG. 13B, the physician (PH) may rotate second lever arm (420b) relative to handle (310) to the position shown in FIG. 11B. As best shown in FIG. 12B, rotation of second lever arm (420b) to the rotational position shown in FIG. 11B actuates plug (426) associated with actuator cable (370) to drive actuator cable (370) proximally. Plug (426) associated with actuator cable (360) may remain unactuated.

Proximal translation of actuator cable (370) drives sliding body (375) proximally within the respective sliding channel (306), while sliding body (365) may remain substantially stationary within sliding channel (306) relative to its position in FIG. 12A. Proximal translation of sliding body (375) drives the section of intermediate portion (372) extending distally from sliding body (375), as well as distal portion (374), proximally. Since distal portion (374) may not actuate proximally out of the distal end of medial segment (320m), as described above, proximal translation of distal portion (374) drives medial segment (320m) to bend to the position shown in FIG. 13B. It will be appreciated that such deflection of medial segment (320m) may result in deflection of end effector (340) and distal segment (320d) in the first direction.

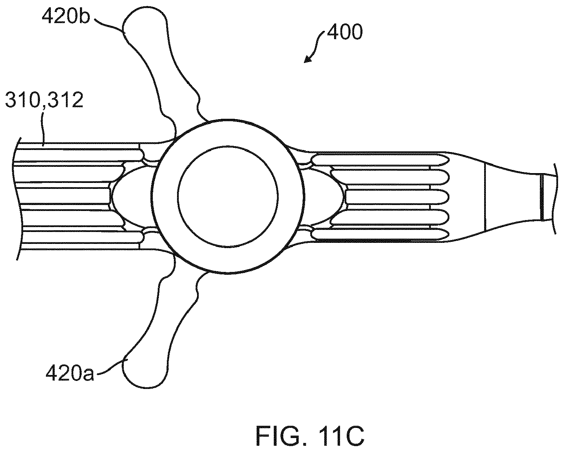

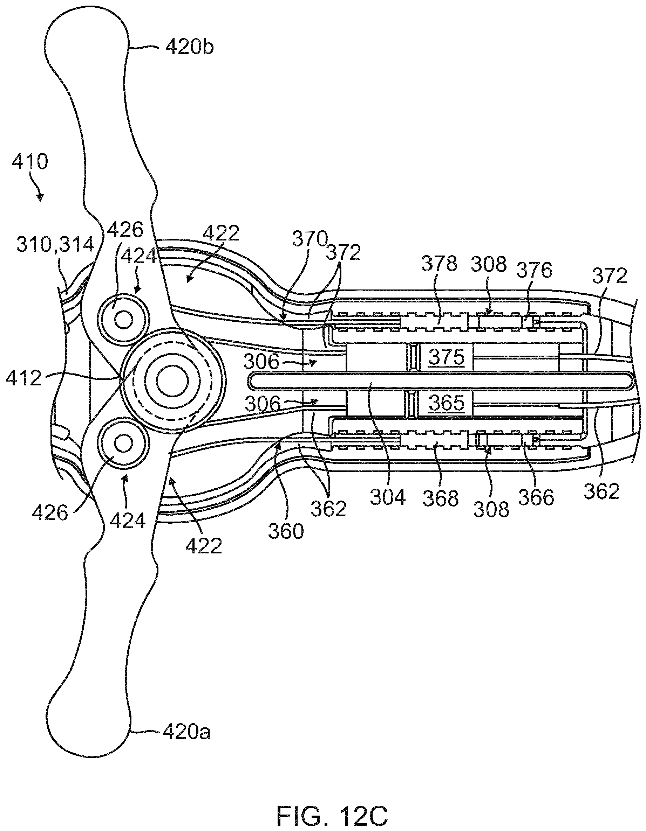

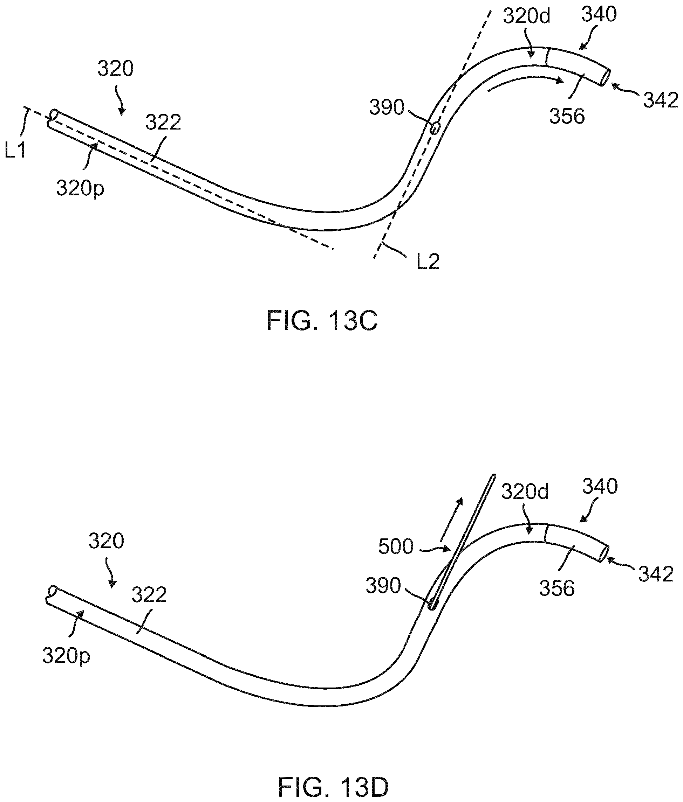

Similarly, when the physician (PH) desires to deflect end effector (340) and distal segment (320d) of catheter (320) in a second direction relative to first and/or second central longitudinal axis (L1, L2) to a deflected state shown in FIG. 13C, the physician (PH) may rotate first lever arm (420a) relative to handle (310) to the position shown in FIG. 11C. As best shown in FIG. 12C, rotation of first lever arm (420a) to the rotational position shown in FIG. 11C actuates plug (426) associated with actuator cable (360) to drive actuator cable (360) proximally. Plug (426) associated with actuator cable (370) may remain actuated.

Proximal translation of actuator cable (360) drives sliding body (365) proximally within sliding channel (306), while sliding body (375) may remain substantially stationary within sliding channel (106) relative to its position in FIG. 12B. Proximal translation of sliding body (365) drives the section of intermediate portion (362) extending distally from sliding body (365), as well as distal portion (364), proximally. Since distal portion (364) may not actuate proximally out of end effector (340), as described above, proximal translation of distal portion (364) drives end effector (340) together with distal segment (320d) to bend to the position shown in FIG. 13C.

As shown in FIG. 13C, when both lever arms (420a, 420b) are rotated away from their respective neutral rotational positions such that both actuator cables (360, 370) are driven proximally, medial segment (320m) and distal segment (320d) of catheter (320) are both in the respective deflected states. More particularly, medial segment (320m) is deflected away from first central longitudinal axis (L1) on a first side of catheter (320) in a first plane and distal segment (320d) is deflected away from second central longitudinal axis (L2) on a second side of catheter (320) in the first plane such that catheter (320) may exhibit asymmetric deflections, for example, a serpentine shape, such as an "S" shape, in the first plane.

It will be appreciated that the particular shape exhibited by catheter (320) when medial segment (320m) and distal segment (320d) of catheter (320) are both deflected may vary from the "S" shape shown in FIG. 13C. In the example shown, medial and distal segments (320m, 320d) are each deflected at generally right angles relative to the respective longitudinal axes (L1, L2). In some versions, medial and distal segments (320m, 320d) may each be only partially deflected, so that medial and distal segments (320m 320d) may each be deflected at generally obtuse angles relative to the respective longitudinal axes (L1, L2) to impart catheter (320) with a less sharp "S" shape than that shown in FIG. 13C. In addition, or alternatively, the particular shape exhibited by catheter (320) when medial segment (320m) and distal segment (320d) of catheter (320) are both fully deflected may be altered by adjusting the locations of tension blocks (368, 378) within adjustment channels (308), the stiffnesses of medial and distal segments (320m, 320d), and/or the range of rotation of lever arms (420a, 420b). For example, such adjustments may limit the range of deflection of medial and distal segments (320m, 320d), so that medial and distal segments (320m 320d) may each be deflected at generally obtuse angles relative to the respective longitudinal axes (L1, L2) to impart catheter (320) with a less sharp "S" shape than that shown in FIG. 13C, or may expand the range of deflection of medial and distal segments (320m, 320d), so that medial and distal segments (320m, 320d) may each be deflected at generally acute angles relative to the respective longitudinal axes (L1, L2) to impart catheter (320) with a more sharp "S" shape than that shown in FIG. 13C.

When medial segment (320m) and distal segment (320d) are in the respective deflected states, catheter (320) may be configured to frictionally engage various portions of an inner surface of one or more anatomical structures, such as lumens. For example, distal segment (320d) may be configured to frictionally engage a corresponding portion of an inner surface of at least one first blood vessel at or near the heart (H) (e.g., the oblique vein of the left atrium) and medial segment (320m) may be configured to frictionally engage a corresponding portion of an inner surface of at least one second blood vessel at or near the heart (H) (e.g., the coronary sinus) when in the respective deflected states. In this manner, medial and distal segments (320m, 320d) may be configured to cooperate with each other when in the respective deflected states to lodge (e.g., wedge) or otherwise anchor catheter (320) against the inner surface(s) of such blood vessel(s), to thereby inhibit inadvertent slipping or other undesired movement of catheter (320) (including end effector (340)) within the heart (H) and thus improve the accuracy of EP mapping or other operations performed using catheter (320), such as tissue ablation. Handle (310) may include any suitable tensioning features for selectively tensioning actuator cables (360, 370) to firmly secure catheter (320) in place, including but not limited to a tensioning knob, etc.

Various other suitable mechanisms that may be used to drive actuator cables (360, 370) independently of each other will be apparent to those skilled in the art in view of the teachings herein.

In some procedures, guidewire (500) may be advanced distally out of inner lumen (392) of catheter (320) through lateral opening (390) while medial segment (320m) and distal segment (320d) are in the respective deflected states, as shown in FIG. 13D. Due to the positioning of lateral opening (390) at or near the distal end of medial segment (320m), guidewire (500) may be deflected together with medial segment (320m); but may not be deflected together with distal segment (320d). As a result, guidewire (500) may be advanced along a pathway that deviates from that along which distal segment (320d) extends, thereby allowing guidewire (500) to access and perform EP mapping or other operations in regions of the heart (H) different from those in which catheter (320) is disposed while catheter (320) remains anchored in place. For example, guidewire (500) may be configured to access at least one blood vessel of the heart (H) (e.g., the great cardiac vein) different from those engaged by medial and distal segments (320m, 320d) (e.g., the oblique vein of the left atrium and the coronary sinus) when advanced distally through lateral opening (390) while medial and distal segments (320m, 320d) are in the respective deflected states.

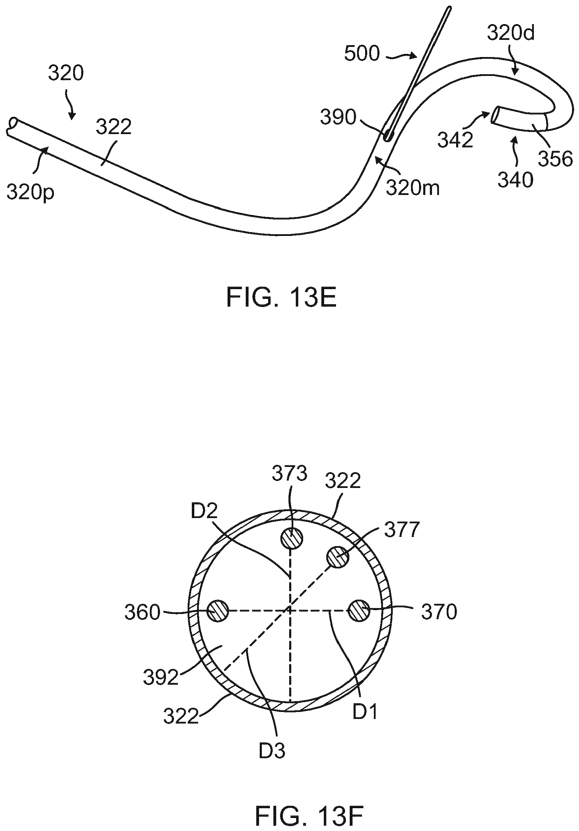

In some procedures, at least a portion of distal segment (320d) may be further deflected in a second plane transverse (e.g., perpendicular) to the first plane such that distal segment (320d) of catheter (320) may exhibit a hook shape, such as a "J" shape, in the second plane as shown in FIG. 13E. For example, deflection drive assembly (400) may include a third actuator cable (not shown) extending along the length of catheter (320) to reach end effector (340), and which may be selectively actuated proximally to drive end effector (340) together with distal segment (320d) to bend to the position shown in FIG. 13E. Handle (310) may include any suitable actuation features for driving such actuation of the third actuator cable, including but not limited to a slider, a pivoting rocker, a dial, etc. Various other suitable mechanisms that may be used to drive such a third actuator cable will be apparent to those skilled in the art in view of the teachings herein. In some embodiments, the first and second actuator cables (360, 370) are situated diametrically opposed to each other on a first diameter (D1) of the lumen (392) of the shaft (322), as shown in FIG. 13F. A third actuator cable 373 may be situated on a different diameter (D2) that is generally perpendicular to the first diameter (D1). Another third or a fourth actuator cable 377 may be situated on a different diameter (D3) not perpendicular to the first diameter (D1). Actuation of the actuator cable 373 enables deflection of the shaft (322) in a plane defined by the diameter (D2) generally perpendicular to the plane defined by the first diameter (D1). Actuation of the actuator cable 377 enables deflection of the shaft (322) in a plane defined by the diameter (D3) angularly offset from the plane defined by the first diameter (D1).

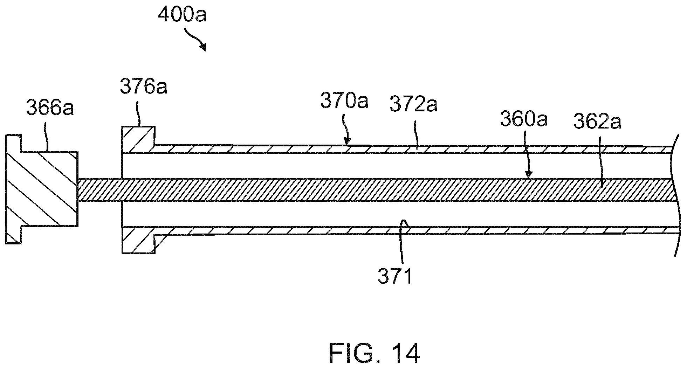

As noted above, other suitable mechanisms may be used to drive first and second actuator cables (360, 370) for deflecting medial segment (320m) and distal segment (320d) of catheter (320). FIG. 14 shows a portion of another example of a deflection drive assembly (400a) including first and second actuator cables (360a, 370a). In the example shown, first and second actuator cables (360a, 370a) include respective intermediary portions (362a, 372a) extending proximally from respective distal portions (not shown) (e.g., through elongate flexible shaft (322) of catheter (320)), and terminating into respective proximal end blocks (366a, 376a). The distal portion of first actuator cable (360a) may be coupled with end effector (340) to prevent actuator cable (360a) from being pulled proximally out of end effector (340), while the distal portion of second actuator cable (370a) may be coupled with the distal end of medial segment (320m) of catheter (320) to prevent actuator cable (370a) from being pulled proximally out of medial segment (320m), in manners similar to those described above. In the example shown, second actuator cable (370a) includes a lumen (371) configured to slidably receive first actuator cable (360a), such that actuator cables (360a, 370a) may be coaxially disposed relative to each other while permitting actuator cables (360a, 370a) to be driven independently of each other. In some versions, a pair of coaxially disposed actuator cables similar to those shown in FIG. 14 may be incorporated into deflection drive assembly (400) in place of actuator cables (360, 370), such as to enable deflection of medial segment (320m) away from first central longitudinal axis (L1) on the second side of catheter (320) in the first plane and deflection of distal segment (320d) away from second central longitudinal axis (L2) on the first side of catheter (320) in the first plane such that catheter (320) may exhibit an inverted "S" shape in the first plane (e.g., a mirror image of the "S" shape shown in FIG. 13C relative to first central longitudinal axis (L1)).

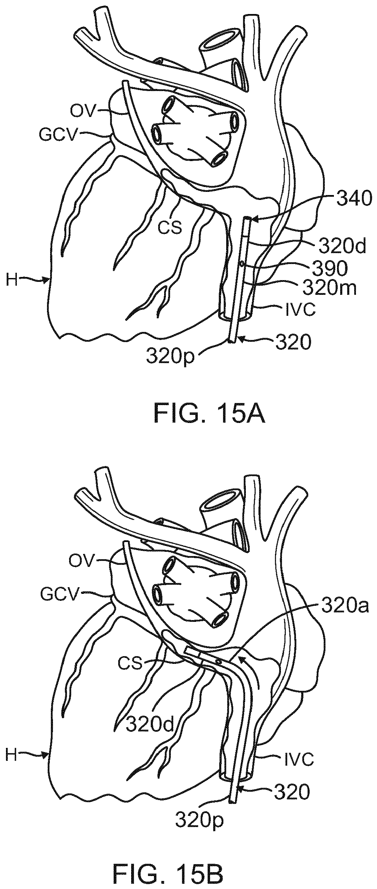

Referring now to FIGS. 15A-15H, in an example of use of catheter assembly (300), catheter (320) may initially be inserted into a major vein or artery (e.g., the femoral vein) of the patient (PA) (e.g., via the leg or groin of the patient (PA)) and then advanced distally along the vein or artery to position medial and distal segments (320m, 320d) of catheter (320) within the inferior vena cava (IVC) of the patient (PA), as shown in FIG. 15A. The physician (PH) may then steer distal segment (320d) into the coronary sinus (CS) of the patient (PA), such as by deflecting medial segment (320m) in the manner described above and/or continuing to advance catheter (320) distally, as shown in FIG. 15B. For example, the physician (PH) may rotate second lever arm (420b) relative to handle (310) to the position shown in FIG. 11B to thereby deflect medial segment (320m) in the first direction, which may result in deflection of distal segment (320d) in the first direction to generally align distal segment (320d) with the coronary sinus (CS). In some procedures, the steering of distal segment (320d) into the coronary sinus (CS) may include generally aligning opening (390) with the great cardiac vein (GCV) of the patient (PA).

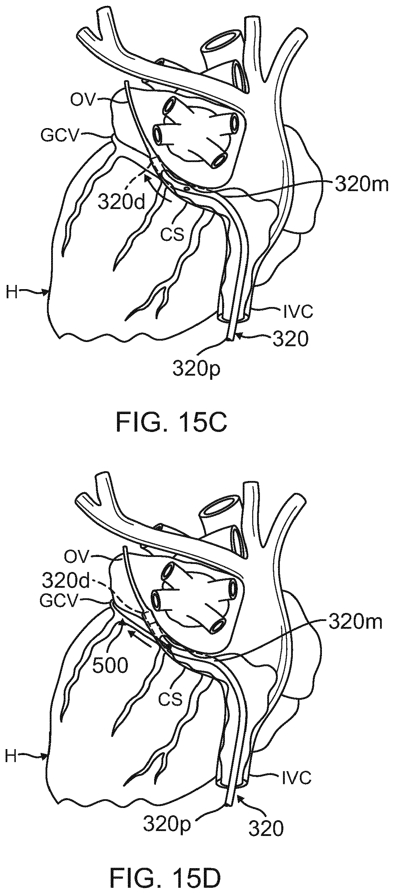

The physician (PH) may then steer distal segment (320d) into the oblique vein of the left atrium (OV) of the patient (PA), such as by deflecting distal segment (320d) in the manner described above and/or continuing to advance catheter (320) distally, as shown in FIG. 15C. For example, the physician (PH) may rotate first lever arm (420a) relative to handle (310) to the position shown in FIG. 11C to thereby deflect distal segment (320d) in the second direction to generally align distal segment (320d) with the oblique vein of the left atrium (OV). In some procedures, the steering of distal segment (320d) into the oblique vein of the left atrium (OV) may include generally aligning opening (390) with the great cardiac vein (GCV). Once distal segment (320d) is suitably positioned within the oblique vein of the left atrium (OV), the physician (PH) may adjust the deflection of one or both segments (320m, 320d), such as by tensioning actuator cables (360, 370), to firmly secure catheter (320) in place, as shown in FIG. 15D. It will be appreciated that distal segment (320d) may frictionally engage the oblique vein of the left atrium (OV) and medial segment (320m) may frictionally engage the coronary sinus (CS) by virtue of being in the respective deflected states to securely anchor catheter (320) within the heart (H). In other words, the positioning of segments (320d, 320m) as shown in FIG. 15D and as described above may effectively provide a mechanical ground for catheter (320) relative to the heart (H).

In some procedures, once catheter (320) is suitably anchored, the physician (PH) may then advance guidewire (500) distally through opening (390) and into the great cardiac vein (GCV), as shown in FIG. 15D. With catheter (320) being anchored/grounded relative to the heart (H) as described above, such anchoring/grounding might prevent slippage of catheter (320) that might otherwise occur when guidewire (500) is advanced into the great cardiac vein (GCV). Guidewire (500) may thus travel along the great cardiac vein (GDV) in a fashion that is much more controlled than would otherwise occur in the absence of catheter (320) being anchored/grounded relative to the heart (H) as described above. With guidewire (500) suitably disposed in the great cardiac vein (GCV), the physician (PH) may then operate guidewire (500) to provide EP mapping, ablation, or any other kind of operations in the great cardiac vein (GCV).

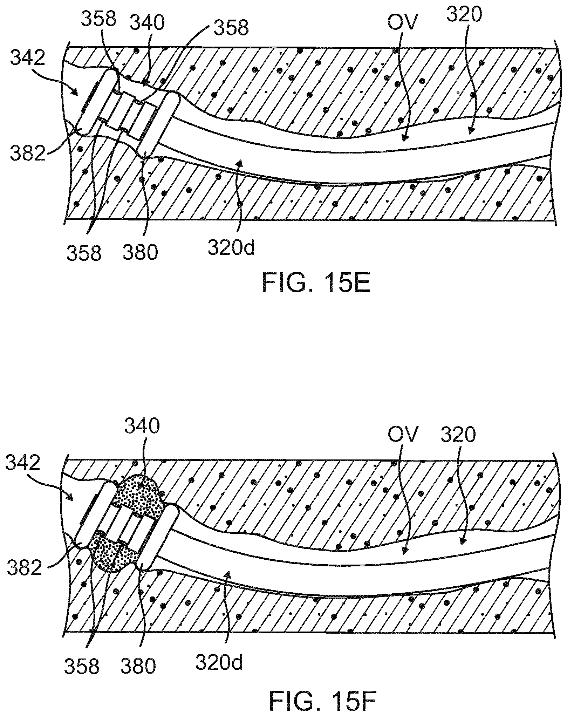

In addition, or alternatively, the physician (PH) may operate catheter (320) to provide EP mapping, ablation, or any other kind of operations in the oblique vein of the left atrium (OV). For example, once catheter (320) is suitably anchored, the physician (PH) may then transition occluding balloons (380, 382) to the respective expanded states, such that occluding balloons (380, 382) sealingly engage corresponding portions of an inner surface of the oblique vein of the left atrium (OV) to fluidly isolate the region of the oblique vein of the left atrium (OV) between occluding balloons (380, 382) from the regions of the oblique vein of the left atrium (OV) beyond occluding balloons (380, 382), as shown in FIG. 15E. Once the region of the oblique vein of the left atrium (OV) between occluding balloons (380, 382) is suitably fluidly isolated, the physician (PH) may then deliver ablative fluid to the fluidly isolated region of the oblique vein of the left atrium (OV) via ablative fluid delivery ports (358) to thereby ablate targeted tissue within the fluidly isolated region, as shown in FIG. 15F.

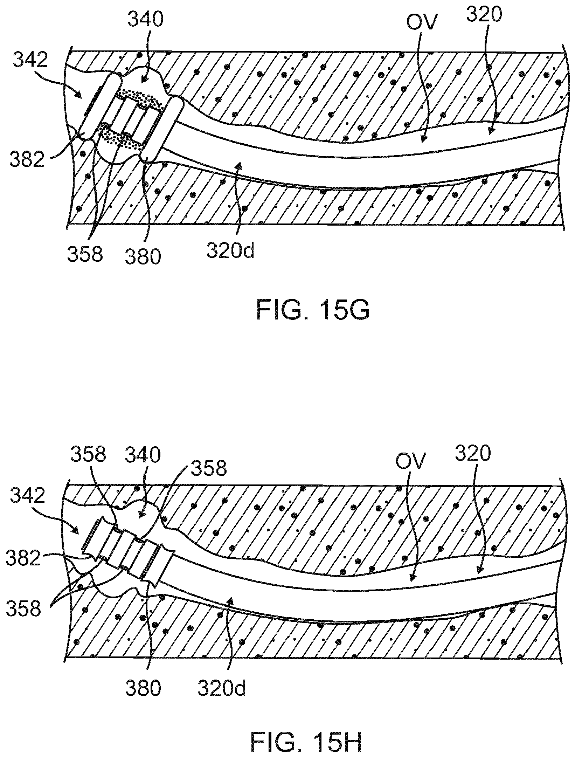

After the targeted tissue has been suitably ablated, the physician (PH) may apply suction to the fluidly isolated region of the oblique vein of the left atrium (OV), such as via ablative fluid delivery ports (358), to evacuate any remaining ablative fluid from the fluidly isolated region, as shown in FIG. 15G. Once the remaining ablative fluid has been suitably evacuated from the fluidly isolated region of the oblique vein of the left atrium (OV), the physician (PH) may then transition occluding balloons (380, 382) to the respective non-expanded states, such that occluding balloons (380, 382) disengage the corresponding portions of the inner surface of the oblique vein of the left atrium (OV), as shown in FIG. 15H. The physician (PH) may then withdraw catheter (320) from the patient (PA).

As noted above, fluid ablation is merely optional, such that other variations of end effector (340) may provide electrical ablation within the oblique vein of the left atrium (OV) via one or more electrodes. Such versions may nevertheless still be positioned as described above with reference to FIG. 15C. As yet another variation, end effector (340) may provide EP mapping within the oblique vein of the left atrium (OV), in addition to or in lieu of providing ablation within the oblique vein of the left atrium (OV). As yet another variation, catheter (320) may lack ablation and/or EP mapping features and capabilities. In some such versions, catheter (320) simply provides a mechanical ground/anchor for guidewire (500) in the heart (H). Some variations of catheter (320) that include ablation and/or EP mapping features and capabilities may omit lateral opening (390) and guidewire (500). Some such variations may nevertheless still have the capability of achieving an "S" shaped curve as described above.

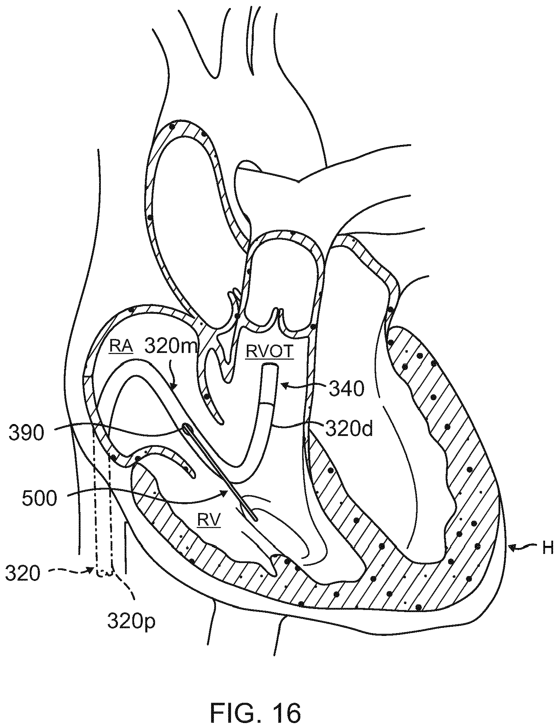

While the foregoing example describes use of catheter (320) in the oblique vein of the left atrium (OV), and guidewire (500) in the great cardiac vein (GCV), catheter (320) and guidewire (500) may alternatively be used in various other anatomical structures. For instance, as shown in FIG. 16, catheter (320) may initially be inserted into a major vein or artery (e.g., the femoral vein) of the patient (PA) (e.g., via the leg or groin of the patient (PA)) and then advanced distally along the vein or artery and/or steered in a manner similar to that described above to position medial segment (320m) of catheter (320) within the right atrium (RA) of the patient (PA) and to position distal segment (320d) of catheter (320) within the right ventricular outflow tract (RVOT) of the patient (PA). As shown, the physician (PH) may advance guidewire (500) distally through opening (390) and into the right ventricle (RV) of the patient (PA). The physician (PH) may then operate guidewire (500) to provide EP mapping, ablation, or any other kind of operations in the right ventricle (RV), and/or may operate catheter (320) to provide EP mapping, ablation, or any other kind of operations in the right ventricular outflow tract (RVOT).

It will be appreciated that the "S" shape of catheter (320), when used to access the right ventricular outflow tract (RVOT) as shown in FIG. 16, may be curved substantially more sharply than the "S" shape of catheter (320) when used to access the oblique vein of the left atrium (OV) as shown in FIGS. 15A-15H. For example, medial and distal segments (320m, 320d) may each be deflected at generally right or obtuse angles relative to the respective longitudinal axes (L1, L2) when used to access the oblique vein of the left atrium (OV); and may each be deflected at generally acute angles relative to the respective longitudinal axes (L1, L2) when used to access the right ventricular outflow tract (RVOT). In some cases, a single catheter (320) may have a range of deflection suitable for performing either operation.

### III. Examples of Combinations

The following examples relate to various non-exhaustive ways in which the teachings herein may be combined or applied. It should be understood that the following examples are not intended to restrict the coverage of any claims that may be presented at any time in this application or in subsequent filings of this application. No disclaimer is intended. The following examples are being provided for nothing more than merely illustrative purposes. It is contemplated that the various teachings herein may be arranged and applied in numerous other ways. It is also contemplated that some variations may omit certain features referred to in the below examples. Therefore, none of the aspects or features referred to below should be deemed critical unless otherwise explicitly indicated as such at a later date by the inventors or by a successor in interest to the inventors. If any claims are presented in this application or in subsequent filings related to this application that include additional features beyond those referred to below, those additional features shall not be presumed to have been added for any reason relating to patentability.

### Example 1

An apparatus, comprising: (a) a body; (b) a catheter extending distally from the body, the catheter including: (i) a proximal segment defining a first longitudinal axis, (ii) a medial segment defining a second longitudinal axis, and (iii) a distal segment; and (c) a deflection assembly, the deflection assembly being configured to deflect the medial segment away from the first longitudinal axis and to deflect the distal segment away from the second longitudinal axis, the deflection assembly including: (i) a first input member associated with the body, (ii) a second input member associated with the body, (iii) a first translating assembly coupled to the medial segment, the first input member being configured to drive the first translating assembly to deflect the medial segment away from the first longitudinal axis, and (iv) a second translating assembly coupled to the distal segment, the second input member being configured to drive the second translating assembly to deflect the distal segment away from the second longitudinal axis.

### Example 2

The apparatus of Example 1, the first and second translating assemblies including first and second actuator cables, respectively.

### Example 3

The apparatus of any of Examples 1 through 2, the first and second input members being configured to drive the first and second translating assemblies, respectively, independently of each other.

### Example 4

The apparatus of any of Examples 1 through 3, the first and second input members including first and second lever arms, respectively, configured to rotate relative to the body about a drive axis.

### Example 5

The apparatus of Example 4, the drive axis being perpendicular with the longitudinal axis.

### Example 6

The apparatus of any of Examples 1 through 5, further comprising an end effector extending distally from the distal segment of the catheter.

### Example 7

The apparatus of Example 6, the end effector including at least one ablative fluid port configured to deliver an ablative fluid to targeted tissue.

### Example 8

The apparatus of Example 7, the end effector including a pair of occluding balloons configured to transition between respective non-expanded and expanded states, the at least one ablative fluid port being longitudinally interposed between the pair of occluding balloons.

### Example 9

The apparatus of any of Examples 6 through 8, the end effector including at least one electrode.

### Example 10

The apparatus of Example 9, the at least one electrode being configured to emit electrical energy.

### Example 11

The apparatus of any of Examples 9 through 10, the at least one electrode being configured to perform electrophysiology mapping.

### Example 12

The apparatus of any of Examples 6 through 11, the end effector including a position sensor.

### Example 13

The apparatus of any of Examples 1 through 12, the catheter including an inner lumen configured to slidably receive a guidewire, the medial segment including an opening configured to direct the guidewire out of the inner lumen.

### Example 14

The apparatus of Example 13, the opening being configured to direct the guidewire out of the inner lumen transversely to the second longitudinal axis.

### Example 15

The apparatus of Example 13, the opening being configured to direct the guidewire out of the inner lumen parallel to the second longitudinal axis.

### Example 16

An apparatus, comprising: (a) a flexible catheter including: (i) a proximal segment defining a first longitudinal axis, (ii) a medial segment defining a second longitudinal axis, and (iii) a distal segment; (b) an end effector extending distally from the distal segment of the catheter, the end effector including at least one ablative fluid port configured to deliver an ablative fluid to targeted tissue; and (c) a deflection assembly, the deflection assembly being configured to deflect the medial segment away from the first longitudinal axis, and to deflect the distal segment and the end effector away from the second longitudinal axis.

### Example 17

The apparatus of Example 16, the end effector including a pair of occluding balloons configured to transition between respective non-expanded and expanded states, the at least one ablative fluid port being longitudinally interposed between the pair of occluding balloons.

### Example 18

An apparatus, comprising: (a) a flexible catheter including: (i) a proximal segment defining a first longitudinal axis, (ii) a medial segment defining a second longitudinal axis, the medial segment including an opening, (iii) a distal segment, and (iv) an inner lumen extending along the proximal and medial segments to the opening; (b) a guidewire slidably disposed in the inner lumen, the opening being configured to direct the guidewire out of the inner lumen; and (c) a deflection assembly, the deflection assembly being configured to deflect the medial segment away from the first longitudinal axis, and to deflect the distal segment away from the second longitudinal axis.

### Example 19

The apparatus of Example 18, the guidewire including at least one electrode.

### Example 20

The apparatus of Example 19, the at least one electrode being configured to perform electrophysiology mapping.

### IV. Miscellaneous

Any of the instruments described herein may be cleaned and sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, hydrogen peroxide, peracetic acid, and vapor phase sterilization, either with or without a gas plasma, or steam.

It should be understood that any of the examples described herein may include various other features in addition to or in lieu of those described above. By way of example only, any of the examples described herein may also include one or more of the various features disclosed in any of the various references that are incorporated by reference herein.

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those skilled in the art in view of the teachings herein. Such modifications and variations are intended to be included within the scope of the claims.

It should be appreciated that any patent, publication, or other disclosure material, in whole or in part, that is said to be incorporated by reference herein is incorporated herein only to the extent that the incorporated material does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

Having shown and described various versions of the present invention, further adaptations of the methods and systems described herein may be accomplished by appropriate modifications by one skilled in the art without departing from the scope of the present invention. Several of such potential modifications have been mentioned, and others will be apparent to those skilled in the art. For instance, the examples, versions, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. An apparatus, comprising:
(a) a housing body;
(b) a catheter shaft extending distally from the housing body, the catheter shaft including:
(i) a proximal segment defining a first longitudinal axis,
(ii) a medial segment defining a second longitudinal axis, and
(iii) a distal segment; and
(c) a deflection assembly configured to deflect the medial segment away from the first longitudinal axis and to deflect the distal segment away from the second longitudinal axis, the deflection assembly including:
(i) a first input member,
(ii) a second input member,
(iii) a first translating assembly coupled to the medial segment, the first input member being configured to drive the first translating assembly to deflect the medial segment away from the first longitudinal axis, and
(iv) a second translating assembly coupled to the distal segment, the second input member being configured to drive the second translating assembly to deflect the distal segment away from the second longitudinal axis.

2. The apparatus of claim 1, the first and second translating assemblies including first and second actuator cables, respectively.

3. The apparatus of claim 1 or claim 2, the first and second input members being configured to drive the first and second translating assemblies, respectively, independently of each other.

4. The apparatus of any preceding claim, the first and second input members including first and second lever arms, respectively, configured to rotate relative to the body about a drive axis., optionally the drive axis being perpendicular with the longitudinal axis.

5. The apparatus of any preceding claim, further comprising an end effector extending distally from the distal segment of the catheter, optionally the end effector including at least one ablative fluid port configured to deliver an ablative fluid to targeted tissue, further optionally wherein the end effector includes a pair of occluding balloons configured to transition between respective non-expanded and expanded states, the at least one ablative fluid port being longitudinally interposed between the pair of occluding balloons.

6. The apparatus of claim 5, the end effector including at least one electrode, optionally the at least one electrode being configured to emit electrical energy.

7. The apparatus of claim 6, the at least one electrode being configured to perform electrophysiology mapping.

8. The apparatus of any of claims 5 to 7, the end effector including a position sensor.

9. The apparatus of any preceding claim, wherein the catheter shaft includes an inner lumen configured to slidably receive a guidewire, and the medial segment includes an opening configured to direct the guidewire out of the inner lumen.

10. The apparatus of claim 9, wherein the opening is (i) configured to direct the guidewire out of the inner lumen transversely to the second longitudinal axis, (ii) configured to direct the guidewire out of the inner lumen parallel to the second longitudinal axis, (iii) formed in a sidewall of the catheter shaft, (iv) formed in a sidewall of the medial segment of the shaft, or (v) includes a distal slope surface configured in a sidewall of the catheter shaft.

11. The apparatus of claim 9, wherein catheter shaft includes a ramp disposed in the opening.

12. The apparatus of claim 11, wherein the ramp (i) extends at an angle relative to the longitudinal axis of the medial segment (ii) includes a Y configuration, (iii) includes first and second divergent prongs, or (iv) includes first and second divergent prongs and the guidewire includes a ring stop configured to engage the first and second divergent prongs.

13. The apparatus of claim 9, wherein the guidewire includes (i) a predetermined pre-bent configuration when the guidewire is in a neutral form, (ii) a shape memory construction, or (iii) a distal segment, a proximal segment and a transverse segment therebetween, the distal segment and the proximal segment being parallel but not coaxial when the guidewire is in neutral form.

14. An apparatus, comprising:
(a) a flexible catheter including:
(i) a proximal segment defining a first longitudinal axis,
(ii) a medial segment defining a second longitudinal axis, and
(iii) a distal segment;
(b) an end effector extending distally from the distal segment of the catheter, the end effector including at least one ablative fluid port configured to deliver an ablative fluid to targeted tissue; and
(c) a deflection assembly configured to deflect the medial segment away from the first longitudinal axis, and to deflect the distal segment and the end effector away from the second longitudinal axis.

15. An apparatus, comprising:
(a) a flexible catheter including:
(i) a proximal segment defining a first longitudinal axis,
(ii) a medial segment defining a second longitudinal axis, the medial segment including an opening,
(iii) a distal segment, and
(iv) an inner lumen extending along the proximal and medial segments to the opening;
(b) a guidewire slidably disposed in the inner lumen, the opening configured to direct the guidewire out of the inner lumen; and
(c) a deflection assembly, the deflection assembly configured to deflect the medial segment away from the first longitudinal axis, and to deflect the distal segment away from the second longitudinal axis.
